(19) Europäisches Patentamt
European Patent Office
Office européen des brevets

(11) EP 1 529 060 B1

(12) # EUROPÄISCHE PATENTSCHRIFT

(45) Veröffentlichungstag und Bekanntmachung des Hinweises auf die Patenterteilung:
**10.09.2014 Patentblatt 2014/37**

(21) Anmeldenummer: **03765097.5**

(22) Anmeldetag: **22.07.2003**

(51) Int Cl.:
*C07K 1/22* (2006.01) *C07K 14/705* (2006.01)
*A61K 39/00* (2006.01) *C07K 16/44* (2006.01)
*C07K 16/30* (2006.01) *C07K 14/47* (2006.01)

(86) Internationale Anmeldenummer:
**PCT/EP2003/008014**

(87) Internationale Veröffentlichungsnummer:
**WO 2004/009632 (29.01.2004 Gazette 2004/05)**

(54) **VERFAHREN ZUR HERSTELLUNG EINES IMMUNSTIMULATORISCHEN MUZINS (MUC1)**

METHOD FOR THE PRODUCTION OF AN IMMUNOSTIMULATING MUCIN (MUC1)

PROCEDE DE PRODUCTION D'UNE MUCINE IMMUNOSTIMULATRICE (MUC1)

(84) Benannte Vertragsstaaten:
**AT BE BG CH CY CZ DE DK EE ES FI FR GB GR HU IE IT LI LU MC NL PT RO SE SI SK TR**

(30) Priorität: **22.07.2002 EP 02016440**

(43) Veröffentlichungstag der Anmeldung:
**11.05.2005 Patentblatt 2005/19**

(73) Patentinhaber: **Glycotope GmbH**
**13125 Berlin (DE)**

(72) Erfinder:
- **GOLETZ, Steffen**
  **16548 Glienicke-Nordbahn (DE)**
- **KARSTEN, Uwe**
  **10437 Berlin (DE)**

(74) Vertreter: **Schiweck, Weinzierl & Koch**
**European Patent Attorneys**
**Landsberger Straße 98**
**80339 München (DE)**

(56) Entgegenhaltungen:
**WO-A-99/34824 DE-A- 10 027 695**
**DE-A- 19 534 630**

- **RYUKO K ET AL: "CHARACTERIZATION OF A NEW MUC1 MONOCLONAL ANTIBODY (VU-2-G7) DIRECTED TO THE GLYCOSYLATED PDTR SEQUENCE OF MUC1" TUMOR BIOLOGY, KARGER, BASEL, CH, Bd. 21, Nr. 4, Juli 2000 (2000-07), Seiten 197-210, XP009022741 ISSN: 1010-4283**
- **KARSTEN U ET AL: "Enhanced binding of antibodies to the DTR motif of MUC1 tandem repeat peptide is mediated by site-specific glycosylation" CANCER RESEARCH, AMERICAN ASSOCIATION FOR CANCER RESEARCH, BALTIMORE, MD, US, Bd. 58, Nr. 12, 15. Juni 1998 (1998-06-15), Seiten 2541-2549, XP002112486 ISSN: 0008-5472**
- **BURCHELL E A: "A short sequence within the amino acidtandem repeat of a cancer associated mucin, contains immunodominant epitopes" INTERNATIONAL JOURNAL OF CANCER, NEW YORK, NY, US, Bd. 44, Nr. 4, 15. Oktober 1989 (1989-10-15), Seiten 691-696, XP002091256 ISSN: 0020-7136**
- **NORUM L F: "Carcinoma-associated MUC1 detected by immunoradiometric assays" TUMOR BIOL, BASEL, CH, Bd. 19 (suppl 1), 1998, Seiten 134-146, XP008026691**
- **VON MENSDORFF-POUILLY S: "Reactivity of natural and induced human antibodies to MUC-1 mucin with MUC1 peptides and n-acetylgalactosamine (GalNac) peptides" INT J CANCER, Bd. 86, 2000, Seiten 702-712, XP002267656**
- **GIMMI ET AL: NAT MED, vol. 2, 1996, pages 1367-1370,**
- **PRICE ET AL TUMOR BIOL Bd. 19, 1998, Seiten 1 - 10**
- **'Product description from Diagnomics', [Online] see letter dated 10-09-2012**
- **'Product description from Gentaur', [Online] see letter dated 10-09-2012**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- 'Invoices for selling antibody A76-A/C7', [Online] see letter dated 10-09-2012
- 'Google search for A76-A/C7', [Online] see letter dated 12-04-2011
- NORUM L F ET AL: 'Carcinoma-associated MUC1 detected by immunoradiometric assays' TUMOUR BIOLOGY : THE JOURNAL OF THE INTERNATIONAL SOCIETY FOR ONCODEVELOPMENTAL BIOLOGY AND MEDICINE 1998 Bd. 19 SUPPL 1, 1998, Seiten 134 - 146 ISSN: 1010-4283
- SNIJDEWINT F G ET AL: 'Cellular and humoral immune responses to MUC1 mucin and tandem-repeat peptides in ovarian cancer patients and controls' CANCER IMMUNOLOGY, IMMUNOTHERAPY : CII APR 1999 Bd. 48, Nr. 1, April 1999, Seiten 47 - 55 ISSN: 0340-7004

SEQUENCE LISTING

<110> Nemod AG

<120> Verfahren zur Herstellung eines immunstimulatorischen MUC1

<130> G 1623 PCT S3

<160> 1

<170> PatentIn version 3.1

<210> 1
<211> 1721
<212> DNA
<213> Homo sapiens

<400> 1

```
gaattccctg gctgcttgaa tctgttctgc cccctcccca cccatttcac caccaccatg      60
acaccgggca cccagtctcc tttcttcctg ctgctgctcc tcacagtgct tacagttgtt     120
acaggttctg gtcatgcaag ctctacccca ggtggagaaa aggagacttc ggctacccag     180
agaagttcag tgcccagctc tactgagaag aatgctgtga gtatgaccag cagcgtactc     240
tccagccaca gccccggttc aggctcctcc accactcagg gacaggatgt cactctggcc     300
ccggccacgg aaccagcttc aggttcagct gccacctggg gacaggatgt cacctcggtc     360
ccagtcacca ggccagccct gggctccacc accccgccag cccacgatgt cacctcagcc     420
ccggacaaca agccagcccc gggctccacc gcccccccag cccacggtgt cacctcggcc     480
ccggacacca ggccgccccc gggctccacc gcccccccag cccacggtgt cacctcggcc     540
ccggacacca ggccgccccc gggctccacc gcgcccgcag cccacggtgt cacctcggcc     600
ccggacacca ggccggcccc gggctccacc gcccccccag cccatggtgt cacctcggcc     660
ccggacaaca ggcccgcctt ggcgtccacc gcccctccag tccacaatgt cacctcggcc     720
tcaggctctg catcaggctc agcttctact ctggtgcaca acggcacctc tgccagggct     780
accacaaccc cagccagcaa gagcactcca ttctcaattc ccagccacca ctctgatact     840
cctaccaccc ttgccagcca tagcaccaag actgatgcca gtagcactca ccatagcacg     900
gtacctcctc tcacctcctc caatcacagc acttctcccc agttgtctac tggggtctct     960
ttctttttcc tgtcttttca catttcaaac ctccagttta attcctctct ggaagatccc    1020
agcaccgact actaccaaga gctgcagaga gacatttctg aaatgttttt gcagatttat    1080

aaacaagggg gttttctggg cctctccaat attaagttca ggccaggatc tgtggtggta    1140
caattgactc tggccttccg agaaggtacc atcaatgtcc acgacgtgga gacacagttc    1200
aatcagtata aaacggaagc agcctctcga tataacctga cgatctcaga cgtcagcgtg    1260
agtgatgtgc catttccttt ctctgcccag tctggggctg ggggtgccagg ctggggcatc   1320
gcgctgctgg tgctggtctg tgttctggtt gcgctggcca ttgtctatct cattgccttg    1380
gctgtctgtc agtgccgccg aaagaactac gggcagctgg acatctttcc agcccgggat    1440
acctaccatc ctatgagcga gtaccccacc taccacaccc atgggcgcta tgtgcccccct   1500
agcagtaccg atcgtagccc ctatgagaag gtttctgcag gtaatggtgg cagcagcctc    1560
tcttacacaa acccagcagt ggcagccact tctgccaact tgtaggggca cgtcgccctc    1620
tgagctgagt ggccagccag tgccattcca ctccactcag ggctctctgg gccagtcctc    1680
ctgggagccc ccaccacaac acttcccagg catggaattc c                         1721
```

**Beschreibung**

Die vorliegende Erfindung betrifft ein Verfahren zur Herstellung eines MUC1-Moleküls, welches in der Lage ist, eine stimulatorische Immunantwort im Menschen hervorzurufen. Die Erfindung betrifft auch ein gereinigtes MUC1-Molekül, das durch die erfindungsgemäßen Verfahren erhältlich ist und im Menschen immunstimulatorisch wirkt. Ein weiterer Gegenstand der vorliegenden Erfindung ist die Verwendung der durch die erfindungsgemäßen Verfahren erhältlichen MUC1-Moleküle zur Herstellung eines Arzneimittels zur Behandlung oder Prävention von Tumoren.

Das humane Muzin MUC1 wurde früher unter anderem auch als Polymorphes Epitheliales Muzin (PEM) bezeichnet. Es ist zur Zeit das einzige Muzin von dem bekannt ist, dass es ein Transmembran-Glykoprotein ist. Das Polypeptid des MUC1 besteht extrazellulär hauptsächlich aus repetitiven Einheiten, sogenannten Tandem-Repeats, deren Anzahl in der humanen Population genetisch bedingt polymorph ist. Entsprechend dem generellen Charakteristikum der Muzine trägt auch MUC1 eine große Menge O-glykosidisch gebundener Kohlenhydratketten. Jedes Tandem-Repeat besteht aus 20 konservierten Aminosäuren (NH2-HGVTSA<u>PDTR</u>PAPGSTAPPA-COOH) mit 5 potentiellen O-Glykosylierungsstellen zwei Serinen und drei Threoninen). Bis vor kurzem war der allgemeine Kenntnisstand der, dass die potentielle Glykosylierungsstelle in der PDTR-Sequenz nicht glykosyliert ist (beispielsweise: Finn OJ, Jerome KR, Henderson RA, Pecher G, Domenech N, Magarian-Blander J, Barratt-Boyes SM. MUC-1 epithelial tumor mucinbased immunity and cancer vaccines. Imm Revs 145:61-89 (1995), p.67). Dies wurde unter anderem darauf begründet, dass nach einer Immunisierung von Mäusen mit MUC1 aus Tumor- oder Nichttumorquellen fast ausschließlich Antikörper erzeugt werden, die gegen die PDTR-Region gerichtet sind, weshalb diese auch als immundominante Region bezeichnet wird. Ein weiterer Grund war, dass in vitro Glykosylierungsversuche zu diesem Zeitpunkt keine Glykosylierung an der PDTR-Region erzielen konnten. Kürzlich wurde jedoch durch vornehmlich massenspektrometrische Untersuchungen von MUC1 aus humaner Milch (Nichttumorquelle) gezeigt, dass das Threonin der PDTR-Region einiger Tandem-Repeats glykosyliert sein kann, andere jedoch unglykosyliert vorliegen. Weiterhin hat die Untersuchung einer Gruppe von MUC1-spezifischen Antikörpern gezeigt, dass eine Glykosylierung in der PDTR-Region die Bindung einzelner Antikörper (Ak) an kurze MUC1-Peptide im Vergleich zum nichtglykosylierten Peptid verstärkt oder erst ermöglicht, während andere völlig unabhängig von der Glykosylierung an dieser Stelle binden.

MUC1 an sich ist ein etablierter Tumormarker. Folgende bekannte Eigenschaften charakterisieren MUC1 als einen Tumormarker: MUC1 wird im normalen Epithel, im wesentlichen Drüsenepithel, ausschließlich polar auf der apikalen Membran der Zellen exprimiert. Diese streng apikale Lokalisation geht während der Kanzerogenese verloren, und es kommt zu einer apolaren Expression über die gesamte Zelloberfläche, wodurch das MUC1 dem Immunsystem zugänglich wird. Eine weitere Eigenschaft des MUC1 ist, dass es während der Kanzerogenese verstärkt exprimiert wird. Als dritte bekannte Tumoreigenschaft ändert sich die Glykosylierung des MUC1 während der Kanzerogenese, wodurch es zu einer aberranten Glykosylierung kommt. Dadurch kommt es zum einen zur Expression von de novo Kohlenhydrat-Antigenen, und zum anderen teilweise zur Freilegung von Peptidepitopen (Karsten, Cancer Research 58, 2541-2549, 1998; Price, Tumor Biol. Suppl.1: 1-5, 1998). MUC1 ist, wie im Stand der Technik beschrieben ein an sich bekanntes Molekül und anerkannt als Tumormarker. Allerdings ist MUC1 kein genau strukturell definiertes Molekül sondern eine heterogene Mischung von Molekülen. Die Heterogenität ist durch verschiedene Eigenschaften von MUC1 begründet:

a. MUC1 ist genetisch polymorph in der humanen Population bezüglich der Anzahl der Tandem Repeats.
b. Die Glykosylierung und Expression ist in unterschiedlichen Geweben und in unterschiedlichen Differenzierungsstadien verschieden.
c. MUC1 Präparationen aus Geweben oder Zelllinien zeigen eine hohe Heterogenität an Kohlenhydratstrukturen und damit an MUC1-Molekülen.
d. MUC1 Präparationen aus Geweben oder Zelllinien zeigen, dass innerhalb eines MUC1-Moleküls die Tandem Repeats heterogen glykosyliert sind, dies bezieht sich auf die Zuckerketten, wie auch auf die Glykosylierungsstellen.
e. Die zur Zeit zur Verfügung stehenden Techniken erlauben keine Zuordnung einer Kohlenhydratkette zu einem bestimmten Tandem Repeat oder einer bestimmten Glykosylierungsstelle von MUC1 aus natürlichem Material.
f. Die Glykosylierung ist zur Zeit für kein MUC1 komplett beschrieben

In keinem Fall ist die chemische Struktur des natürlichen MUC1-Moleküls komplett bekannt. Im Gegenteil, die Glykosylierung ist in wenigen Fällen und dann nur partiell bekannt. Das zeigt, dass es sich bei herkömmlichen natürlichen MUC1 um keine definierte Struktur handelt, sondern um eine große heterogene Gruppe an Molekülen.

Hinzu kommt, dass im Tumorfall durch die Veränderungen im Glykosylierungsapparat Veränderungen der MUC1-Glykosylierung auftreten, die eine zusätzliche Komplexität in die Heterogenität einführen. Auch diese veränderte Glykosylierung ist stark heterogen in Bezug auf Tumorart, innerhalb einer Tumorart, bezüglich Kohlenhydratketten, Verteilung der Kohlenhydratketten.

Es ist beschrieben, daß tumorassoziiertes MUC1 immunsuppressiv auf die T-Zell Proliferation wirkt. (Agrawal et al., Nat. Med., 1998, Oct. 4(10):1093). Weiter wird beschrieben, dass es sich dabei im Besonderen um längere MUC1-Moleküle mit mehr als 60 Aminosäuren handelt, die aus Tumorzellen gewonnen werden (WO 99/23114). Eine entsprechende Immunsuppression ist äußerst nachteilhaft oder verhindert den Gebrauch von entsprechenden tumorassoziierten MUC1-Molekülen, die aus Zellen gewonnen wurden. Im Besonderen wurde beschrieben, daß MUC1, das mit Hilfe des Antikörpers B27.29 gewonnen wurde, immunsuppressiv wirkt (US 20020044943, WO 9810783). Für eine geeignete und wirksame Tumortherapie oder Prophylaxe auf Basis des Tumormarkers MUC1 ist es notwendig eine Möglichkeit zu finden MUC1-Moleküle herzustellen, die nicht oder vermindert immunsuppressiv und im bevorzugten Fall sogar immunstimulatorisch wirken. Eine etablierte Tumortherapie oder Prophylaxe auf Basis des Tumormarkers MUC1 ist nicht bekannt.

DE 100 27 695 beschreibt Vakzine gegen konformationsabhängige Antigene, wie z.B. anti-Idiotypische Antikörper gegen MUC1 Konformationsepitope. Hierzu werden anti-MUC1 Antikörper als Antigen zum Durchmustern einer Phagenbibliothek verwendet.

Ryuko et al. (2000), Tumor Biol 21:197-210 beschreibt glykosylierte MUC1-Peptide, die von verschiedenen anti-MUC1-Antikörpern gebunden werden. Diese MUC1-Peptide sind nicht als im Mensch eine stimulatorische Immunantwort hervorrufend beschrieben und zum anderen sind es Peptide

DE 195 34 630 beschreibt die Verwendung des A76-A/C7 Antikörpers zur Präparation von MUC1 aus Brusttumorzellen, Pankreasepithel oder kolorektalen Karzinomen, siehe Spalte 3, 23-26. Die so isolierten Moleküle sind nicht als immunstimulatorisch wirkend beschrieben.

Snijdewint et al. (1999), Cancer Immunol. Immunther 48:47-55 beschreibt die Gewinnung von MUC1 aus dem Überstand von ZR-75-1 Zellen mittels Filtration und mittels des Antikörpers 139H2. Das so gewonnene MUC1-Molekül wirkt, wie in Abbildung 4 von **D4** gezeigt, immunsupprimierend.

Price et al. (1998), Tumor Biol 19 (suppl I):1-20 beschreibt die Charakterisierung von 56 monoklonalen Antikörpern gegen MUC1. Dabei wird auch eine MUC1 enthaltende Präparation der Brustkrebs-Zellllinie ZR-75 verwendet (siehe Seite 5, rechte Spalte). **D5** beschreibt dabei nicht, wie die MUC1 enthaltende Präparation von ZR-75 Zellen hergestellt wurde.

Gimmi et al. (1996), Nat. Med 2(12):1367-1370 beschreibt die Präparation von MUC-1 aus dem Überstand von ZR-75.1 Zellen mittels eines sogenannten anti-DF3 Antikörpers. Das so isolierte MUC1 wirkt immunsupprimierend (siehe Seite 1368, Abbildung 1a).

Das technische Problem, das der Erfindung zugrunde liegt, ist somit die Bereitstellung von Maßnahmen zur Therapie und/oder Prophylaxe von Tumoren, die mit dem Tumormarker MUC-1 assoziiert sind.

Dieses technische Problem wird durch die in den Ansprüchen gekennzeichneten Ausführungsformen gelöst.

Die vorliegende Erfindung betrifft daher ein Verfahren zur Herstellung eines MUC1-Moleküls, welches in der Lage ist eine stimulatorische Immunantwort im Menschen hervorzurufen, umfassend:

a) in Kontakt bringen eines Gemisches von MUC1-Molekülen aus dem Überstand von ZR-75-1 Zellen mit dem Antikörper A76-A/C7
für einen Zeitraum der ausreichend ist und unter Bedingungen die geeignet sind, damit sich ein Immunkomplex ausbildet;
b) Isolierung des Immunkomplexes; und
c) Bereitstellung des MUC1-Moleküls aus dem Immunkomplex.

Der Begriff "Herstellung" umfasst neben den zuvor explizit genannten Schritten auch zusätzliche Schritte, wie etwa Vorbehandlungen des Ausgangsstoffes oder Weiterbehandlungen des Endproduktes. Vorbehandlungsverfahren werden nachfolgend noch genauer erläutert, Weiterbehandlungsverfahren umfassen beispielsweise die endgültige Formulierung des hergestellten MUC1-Moleküls in geeigneten Darreichungsformen. Die Art der Darreichungsform, z.B. Tablette, Lösung oder Lyophilisat, hängt hierbei von der beabsichtigten Verwendung ab. Dem Fachmann ist hierbei bekannt, welche Darreichungsform sich für welchen Verwendungszweck eignet. Je nach Darreichungsform kann das durch das erfindungsgemäße Verfahren hergestellte MUC1-Molekül zusammen mit Hilfs-, Träger-, oder weiteren Wirkstoffen vorliegen. Hilfsstoffe sind hierbei vorzugsweise Adjuvantien, weitere Wirkstoffe vorzugsweise immunstimulatorische Moleküle, wie Interleukine. Das mit dem erfindungsgemäßen Verfahren hergestellte MUC1-Molekül kann auch in Weiterbehandlungsschritten chemisch modifiziert werden. Vorzugsweise wird das MUC1-Molekül hierbei mit einem oder mehreren weiteren Molekülen in geeigneter Weise, d.h. durch chemische oder physikalische Interaktion, verbunden. Als weitere Moleküle im Sinne der Erfindung dienen bevorzugt andere Proteine oder Peptide, die mit dem durch das erfindungsgemäße Verfahren hergestellten MUC1-Molekül fusioniert werden. Hierbei liegt das MUC1-Molekül dann als Fusionsprotein vor. Als weitere Moleküle im Rahmen des erfindungsgemäßen Verfahrens sind besonders Substanzen geeignet, die eine therapeutische oder diagnostische Wirkung entfalten, beispielsweise Radioisotope oder Toxine. Ebenso fallen als weitere Moleküle solche Substanzen unter die Erfindung, die geeignet sind, als Trägermaterialien zu dienen oder die als Kopplungskomponenten für die MUC1-Moleküle mit Trägern dienen können. Solche Trägermaterialien oder Kopplungskomponenten sind im Stand der Technik bekannt.

Der Begriff "MUC1-Molekül, welches in der Lage ist eine Immunantwort im Menschen hervorzurufen", umfasst MUC1-Moleküle oder Fragmente davon, die zu einer Spezies von MUC1-Glykoproteinen gehören, die alleine oder in Kombination mit geeigneten Adjuvantien und/ oder immunstimulatorischen Molekülen eine Immunantwort gegen MUC1-positive Tumorzellen bewirken. Unter Immunantwort versteht man im Sinne der Erfindung das Auslösen einer humoralen Immunantwort und/ oder zellulären Immunantwort. Unter einer humoralen Immunantwort versteht man dabei die Induktion von Antikörpern durch die mit dem erfindungsgemäßen Verfahren hergestellte MUC1-Moleküle. Unter einer zellulären Immunantwort versteht man dabei die Induktion von Immunzellen, beispielsweise CD4 positive und/oder CD8 positive Zellen, durch die mit dem erfindungsgemäßen Verfahren hergestellte MUC1-Moleküle, die eine Bekämpfung von MUC1-positiven Tumorzellen direkt oder indirekt bewirken. Die MUC1-Moleküle zeichnen sich dadurch aus, dass sie nicht oder nur schwach immunsuppressiv auf T-Zellen wirken. Bevorzugt werden dabei MUC1-Moleküle, die T-Zellen stimulieren, d.h. diese Moleküle wirken im Sinn der vorliegenden Erfindung stimulatorisch. Die MUC1-Moleküle sind vorzugsweise tumorassoziiert. Die immunstimulatorischen Eigenschaften von MUC1-Molekülen und induzierten Immunantworten können durch mindestens eines der in den Beispielen beschriebenen Verfahren oder andere dem Fachmann bekannte Verfahren in vitro und in vivo nachgewiesen werden, beispielsweise ELISA, ADCC- und CDC-Assays, T Zell-Stimulierungs-Assays und NOD-SCIDMaus-Modelle. Dem Fachmann ist es daher ohne weiteres möglich zu bestimmen, ob bestimmte MUC1-Moleküle zu derselben Spezies hinsichtlich ihrer immunstimulatorischen Eigenschaften gehören. Vorzugsweise teilen die Mitglieder dieser Spezies in einem oder mehreren Tandem Repeat(s) chemische Eigenschaften, die nachfolgend genauer definiert werden.

Unter dem Begriff "in Kontakt-Bringen" sind alle Maßnahmen zu verstehen, durch die eine chemische, biochemische oder physikalische Interaktion zwischen dem MUC1-Molekül und den im Verfahren eingesetzten Antikörpern ermöglicht wird. Die MUC1-Moleküle und die Antikörper werden hierzu vorzugsweise in eine geeignete Flüssigkeit eingebracht. Als geeignete Flüssigkeit dienen beispielsweise Pufferlösungen, die, wie im Stand der Technik oder den Beispielen beschrieben, in ELISA- oder RIA-Nachweissystemen eingesetzt werden oder Lösungen, die für chromatographische Reinigungsverfahren beschrieben wurden. Besonders geeignet sind Lösungen, die zur Affinitätschromatographie mit Antikörpern im Stand der Technik eingesetzt wurden. Geeignete Lösungen sind ganz besonders bevorzugt die in den Beispielen genauer beschriebenen Lösungen.

Der Begriff "Gemisch von MUC1-Molekülen" umfasst eine Vielzahl von MUC1-Molekülen, die derselben oder unterschiedlichen Spezies angehören können. Wie bereits zuvor ausgeführt wurde, handelt es sich bei dem im Stand der Technik beschriebenen MUC1-Tumormarker um ein Gemisch von unterschiedlichen molekularen Spezies, die sich hinsichtlich ihrer Glykosylierung und teilweise in ihrer Aminosäurezusammensetzung unterscheiden. Das im erfindungsgemäßen Verfahren eingesetzte Gemisch von MUC1-Molekülen kann neben diesen auch weiter Moleküle enthalten, beispielsweise Zucker, Lipide oder Proteine oder Peptide, die nicht zur MUC1-Gruppe gehören. Zum Beispiel können solche Gemische von MUC1-Molekülen aus Tumorgeweben, Tumorzellen sowie aus Zellen oder Zelllinien, die, inter alia, tumorassoziiertes MUC1 produzieren gewonnen werden. Beispielsweise können aus diesen Geweben, Zellen oder Zeillinien Lysate hergestellt werden. Diese Zelllysate oder Teile davon können auch als MUC1-Gemische dienen. Als Gemisch von MUC1-Molekülen können auch synthetische Glykopeptide oder Fragmente des tumorassoziierten MUC1 dienen. Die Herstellung von synthetischen Glykopeptiden ist im Stand der Technik bekannt (Karsten, loc cit.). Glykopeptide können darüber hinaus durch Behandlung mit Proteasen und/ oder Glykosidasen aus MUC1-Präparationen hergestellt werden. Ganz besonders bevorzugt ist der Überstand von ZR-75-1 Zellen, der in dem erfindungsgemäßen Verfahren verwendet wird

Der Begriff "Antikörper" umfasst im Sinne der Erfindung murine, chimäre, humanisierte, humane Antikörper, ganze Antikörper verschiedener Isotypen oder Antikörperfragmente, beispielsweise single-chain Antikörperfragmente (ScFv), Fab-Fragmente, multivalente Antikörperfragmente (beispielsweise Dia-, Tria-, oder Tetrabodies), oder Antikörperfragmente anderer Formate, auch solche, die, als Fusionsproteine mit anderen Peptiden oder Proteinstrukturen gekoppelt, verwendet werden, sowie Antikörper oder Antikörperfragmente, die neben der erfindungsgemäßen Spezifität für MUC1 noch eine oder mehrere weitere Spezifitäten besitzen, beispielsweise bispezifische Antikörper. Die erfindungsgemäße Spezifität der erfindungsgemäßen Antikörper ist weiter unten ausgeführt. Die Herstellung dieser Antikörper erfolgt nach an sich bekannten Techniken. Die in den erfindungsgemäßen Verfahren eingesetzten Antikörper zeichnen sich durch die zuvor genannten Eigenschaften aus.

In diesem Zusammenhang versteht man unter dem Begriff "immundominante Region des MUC1-Tandem Repeats", im Sinne der Erfindung die PDTR-Region, die streng genommen weitere Aminosäuren der umliegenden Sequenzen des MUC1-Tandem Repeats beinhaltet, beispielsweise APDTRPAP. Ob ein Antikörper an diese Region bindet kann durch im Stand der Technik bekannte Verfahren ohne weiteres vom Fachmann bestimmt werden.

Der Begriff "MUC1-Tandem Repeat" bezeichnet eine Sequenz aus 20 Aminosäuren: HGVTSAPDTRPAPGSTAPPA. Die fünf potentiellen O-Glykosylierungsstellen an zwei Serinen und drei Threoninen sind in der zuvor gezeigten Sequenz unterstrichen. Darüber hinaus erfasst dieser Begriff erfindungsgemäß auch solche Varianten, die Mutationen und damit Aminosäureaustausche in einem oder mehrerer Tandem Repeats eines längeren MUC1-Moleküls besitzen. Das Tandem Repeat besitzt 20 Aminosäuren und kann an beliebigen Stellen beginnen.

Der Begriff "nicht-glykosylierte MUC1-Tandem Repeats in Serie" bezeichnet eine lineare Abfolge von mehreren Tandem Repeats des MUC1, wobei die Abfolge nicht durch andere Sequenzen unterbrochen ist. Vorzugsweise liegen mindestens zwei" mindestens drei, mindestens vier oder mindestens fünf Tandem Repeats in Serie vor. Am meisten bevorzugt sind mindestens sechs Tandem Repeats in Serie.

Unter dem Begriff "erhöht" ist im Sinne der Erfindung eine Bindung des Antikörpers gegenüber eines PDTR-glykolisierten MUC1-Peptides zwischen 9 und 40 Aminosäuren zu verstehen, die signifikant stärker ist, als die Bindung gegen ein entsprechendes Peptid, das nicht an der PDTR-Region glykosyliert ist.. Dies gilt für PDTR-glykosylierte Tandem Repeats unter dem bereits ausgeführten Punkt ii (ii - Bindung an ein MUC1-Fragment, welches zwischen 9 und 40 Aminosäuren lang ist und Sequenzen des MUC1-Tandem Repeats und der immundominanten Region enthält, wird durch Glykosylierung des Threonins einer PDTR-Sequenz ermöglicht oder erhöht). Man versteht darunter weiterhin eine Bindung des Antikörpers gegenüber mehreren Tandem Repeats in Folge, die signifikant stärker ist, als eine Bindung gegen einzelne Tandem Repeats. Dies gilt für nicht glykosylierte Tandem Repeats unter dem Punkt iii) Bindung an nicht-glykosylierte MUC1-Tandem Repeats wird erhöht, wenn die nicht glykosylierten MUC1-Tandem Repeats in Serie vorliegen;) und gegenüber PDTR-glykosylierten Tandem Repeats unter dem Punkt iv) (iv - Bindung an multiple glykosylierte MUC1-Tandem Repeats, die Glykosylierungen an den Threoninen mehrerer PDTR-Sequenzen der immundominanten Region tragen, ist gemäß eines addditiven Effektes aus Länge und PDTR-Glykosylierung gegenüber kurzen MUC1-Fragmenten aus ii) erhöht). Ob eine Bindung signifikant erhöht ist, kann durch geeignete statistische Tests vom Fachmann leicht bestimmt werden und wird in den Beispielen 1 bis 3 veranschaulicht.

Unter dem Begriff "ermöglicht" ist im Sinne der Erfindung eine Bindung eines Antikörpers gegenüber eines PDTR-glykosylierten MUC1-Peptides zwischen 9 und 40 Aminosäuren, die durch die Glykosylierung an dieser Stelle ermöglicht wird, zu verstehen. Entsprechende Peptide, die nicht an der PDTR-Region glykosyliert sind, binden nicht oder die Bindung ist mit den verwendeten Methoden kaum detektierbar (siehe auch 2).

Unter dem Begriff "Immunkomplex" ist im Sinne der Erfindung das Produkt einer Antikörper-Antigen-Reaktion zu verstehen. Hierbei bindet der Antikörper über physiko-chemische Interaktionen reversibel an das Antigen. Die interagierenden Komponenten bilden den Immunkomplex.

Der Begriff "Isolierung" umfasst alle Maßnahmen oder Verfahren, die dazu dienen, den Immunkomplex von Antikörpern abzutrennen, die keinen Immunkomplex mit einem Antigen gebildet haben sowie von MUC1-Molekülen oder anderen Molekülen, die nicht von einem Antikörper gebunden wurden. Diese Verfahren sind im Stand der Technik bekannt und in den Beispielen beschrieben und umfassen beispielsweise Maßnahmen, die zur Ausfällung des Immunkomplexes führen oder chromatographische Verfahren. Weitere geeignete Verfahren werden auch nachfolgend noch genauer beschrieben.

Darüber hinaus wird hierin ein neuartiges und unerwartetes Tumorepitop auf dem Gesamtmolekül von MUC1 beschrieben, das durch das erfindungsgemäße Verfahren gewonnen werden kann. Das neuartige MUC1-Tumorepitop auf dem Gesamtmolekül von MUC1 ist charakterisiert durch die gemeinsamen Bindungsspezifitäten von MUC1-spezifischen monoklonalen Antikörpern der Gruppe 1, die im Folgenden im Detail beschrieben werden. Alle untersuchten monoklonalen Antikörper, welche die im folgenden beschriebenen gemeinsamen Bindungseigenschaften besitzen, wurden durch Immunisierung von Mäusen mit MUC1 aus Tumormaterialien generiert:

Antikörper der Gruppe 1 haben die gemeinsam zutreffenden Bindungseigenschaften 1 bis 4:

1) Die Antikörper binden die immundominante Region (sogenannte PDTR-Region - siehe Definition) des MUC1-Tandem Repeats.

2) Die Bindung der Antikörper an ein MUC1-Fragment der MUC1-Tandem Repeats, das die PDTR-Region enthält und zwischen 9 und 40 Aminosäuren lang ist, wird durch eine Glykosylierung an dem Threonin einer PDTR Sequenz der immundominanten Region ermöglicht oder erhöht. Die Bindung an die entsprechenden MUC1-Peptide, die nicht in der PDTR Sequenz glykosyliert sind, ist deutlich niedriger. Eine Glykosylierung mit Tn (GalNAc$\alpha$1-O-Thr) ist dabei ausreichend um diesen Effekt zu erreichen, allerdings ist auch eine längere Glykosylierung wie beispielsweise durch TF (Gal$\beta$1-3GalNAc$\alpha$1-O-Thr) möglich um den Effekt zu erreichen. Glykosylierungen an anderen Glykosylierungsstellen des MUC1-Tandem Repeats haben keinen oder keinen nennenswerten Einfluß auf den Grundeffekt (siehe Beispiel 1 und Tabelle 1)

3) Die Antiköper binden an nicht-glykosylierte MUC1 Tandem-Repeats besser, wenn sie in Serie vorliegen. Dabei enthält das multiple nichtglykosylierte MUC1 Tandem-Repeat-Peptid mindestens 3 Tandem-Repeats, wobei die Verstärkung der Bindung auch erst durch weitere Tandem Repeats zustande kommen kann und/oder durch eine erhöhte Anzahl an Tandem Repeats noch deutlich stärker erhöht wird. Bei den meisten Antikörpern ist die größte Bindungszunahme von MUC1-Peptiden mit 4 Tandem Repeats nach MUC1-Peptiden mit 6 Tandem Repeats zu beobachten (siehe Beispiel 2 und Tabelle 2).

4) Die Antikörper zeigen eine Bindungseigenschaft, die erfindungsgemäß als additiver Effekt bezeichnet wird. Die Antiköper binden an PDTR-glykosylierte MUC1-Tandem-Repeats besser, wenn sie in Serie vorliegen. Dabei enthält das multiple PDTR-glykosylierte MUC1-Tandem-Repeat-Peptid mindestens 2 Tandem-Repeats und mindestens 2 glykosylierte PDTR-Regionen. Die Verstärkung der Bindung kann auch erst durch weitere PDTR-glykosylierte Tandem-Repeats zustande kommen und/oder durch eine erhöhte Anzahl an PDTR-glykosylierten Tandem-Repeats noch deutlich stärker erhöht werden. Bei vielen Antikörpern ist die größte Bindungszunahme für multiple PDTR-glykosylierte MUC1-Tandem-Repeat-Peptiden von 4 Tandem Repeats zu solchen mit 6 Tandem Repeats zu beobachten (siehe Beispiel 3 und Tabelle 3).

Das Tumorepitop wird durch die Bindungsspezifitäten der Antikörper beschrieben, die die Bindungseigenschaften 1 und 2 und 3 und 4 aufweisen. Bevorzugt ist dabei die antigene Epitopstruktur, die durch die Antikörper mit besonders starkem additiven Effekt gebunden wird, beispielsweise A76-A/C7 und VU-11 E2 und dabei bevorzugt A76-A/C7. Eine weiter bevorzugte Form des erfindungsgemäßen MUC1-Tumorepitopes ist die Struktur, die bevorzugt durch die Antikörper mit einem besonders starken additiven Bindungseffekt, beispielsweise A76-A/C7, VU-11E2, und dabei im besonderen durch A76-A/C7, gebunden wird, da A76-A/C7 einer stark verminderte Bindung zu in Serum abgegebenes MUC1 ("gesheddetes" MUC1) in Kolonkarzinompatienten aufweist. Im Gegensatz dazu zeigt HMFG-1 eine deutlich stärkere Bindung in Kolon- und Pankreaskarzinompatienten. Dieser Fakt ist ein großer Vorteil für Immuntherapien, die MUC1-Moleküle oder MUC1-spezifische Antikörper mit dieser Spezifität verwenden

Da der Längeneffekt von Antikörpern gegenüber nicht-glykosylierten Tandem Repeats allein keine Differenzierung zwischen Antikörpern, die durch Immunsierung mit Tumormaterial, und solchen Antikörpern, die durch eine Immunisierung mit Nicht-Tumormaterial hergestellt wurden erreicht, zeigt, wird deutlich, dass die in der Literatur beschriebene Loop-Struktur, die in nicht-glykosylierten MUC1-Peptiden multipler Tandem Repeats auftritt, nicht exakt in dieser dort beschriebenen Form dem erfindungsgemäßen Tumorepitop entspricht.

Alle getesteten Antikörper aus Immunisierungen mit MUC1 aus Tumormaterial zeigen eine Bindungseigenschaft, die abhängig von der Glykosylierung der PDTR-Region ist, bevorzugt ist dabei eine MUC1-Struktur die mindestens 9 Aminosäuren lang ist und eine Umgebung von mindestens 3 zusätzlichen Aminosäuren um die PDTR Region beinhaltet, und davon, dass nicht-glykosylierte MUC1-Tandem Repeats in Serie vorliegen. Der Großteil zeigt den additiven Bindungseffekt aus multiplen Tandem Repeats und PDTR-Glykosylierung.

Demgegenüber sind fast alle getesteten Antikörper, die durch Immunisierung mit Nicht-Tumormaterial gewonnen wurden, glykosylierungsunabhängig. Die meisten Antikörper sind negativ für den additiven Effekt.

Bevorzugte Antikörper für die erfindungsgemäße Definition und Beschreibung des Tumorepitops und zur Anwendung dieser Antikörper für Tumordiagnostik und -behandlung sowie zur erfindungsgemäßen Gewinnung von geeignetem MUC1-Molekülen zur Tumorbehandlung und -diagnostik, sind solche Antikörper aus Gruppe1 die einen additiven Bindungseffekt aus Länge und PDTR-Glykosylierung aufweisen, wie er unter Bindungseigenschaft 4 beschrieben wurde, beispielsweise A76-A/C7, VU-11E2, VU-11D1, BC4E549, VU-12E1, VU-3D1 und b-12 (siehe Tabelle 3; Quelle der Antikörper ist in Tabelle 1 beschrieben). Diese Antikörper weisen eine erhöhte Affinität gegenüber multiplen Tandem Repeats und multiplen PDTR-glykosylierten Tandem Repeats und damit gegenüber nativen Tumor-MUC1, das aus multiplen Tandem Repeats besteht, auf und sind daher erfindungsgemäß zu bevorzugen.

Besonders bevorzugt sind Antikörper für die erfindungsgemäße Definition und Beschreibung des Tumorepitops und zur Anwendung dieser Antikörper für Tumordiagnostik und Behandlung sowie zur erfindungsgemäßen Gewinnung von geeignetem MUC1-Molekülen zur Tumorbehandlung und-diagnostik, die einen starken additiven Bindungseffekt aufweisen. Diese Antikörper binden an multiple PDTR-glykosylierte MUC1-Tandem-Repeat-Peptide aus 4) deutlich bis vielfach stärker als an kurze PDTR-glykosylierte MUC1-Peptide aus 2) und ihre Bindung an multiple nichtglykosylierte MUC1-Tandem Repeats aus 3). Die Antikörper dieser bevorzugten Untergruppe zeigen einen starken additiven Effekt der Bindungsverstärkung durch Glykosylierung der PDTR-Sequenz nach 2) und Multimerisierung der Tandem-Repeats nach 3) (siehe Beispiel 3 und Tabelle 3). Die Antikörper dieser Gruppe, vorzugsweise A76-A/C7, VU-11E2 und VU-11D1, (siehe Beispiel 3 und Tabelle 3), zeigen eine besonders starken additiven Effekt und weisen eine besonders erhöhte Affinität gegenüber multiplen PDTR-glykosylierten Tandem Repeats und damit gegenüber nativem Tumor-MUC1, das aus multiplen Tandem Repeats besteht, auf und sind daher erfindungsgemäß zu bevorzugen.

Alle getesteten MUC1-spezifischen Antikörper, die Bindungseigenschaften der Gruppe 1 besitzen, korrelieren mit dem Immunogen, das zu ihrer Erzeugung in Mäusen verwendet wurde (siehe Beispiel 2 und Tabelle 1), beispielsweise in Form ganzer Tumorzellen oder aus Tumormaterial gewonnenem MUC1. MUC1-spezifische monoklonale Antikörper (mAK), die aus Immunisierungen mit nicht tumorrelevantem MUC1-Material gewonnen wurden zeigen diese Kombination an Bindungseigenschaften nicht. Sie sind alle unabhängig oder invers (negativ) abhängig von der Glykosylierung der PDTR-Region nach 2) oder unabhängig von der Länge der unglykosylierten MUC1-Peptide und damit der Zahl der Tandem Repeats nach 3). Diese unerwartete Korrelation zeigt, dass Antikörper, die die Bindungseigenschaften der Gruppe 1 besitzen, eine besondere antigene Struktur auf dem MUC1 erkennen, die einem neuartigen Tumorepitop auf MUC1 entspricht und damit als Zielstruktur für eine Tumortherapie und für diagnostische Zwecke besonders gut geeignet ist. Therapeutische und diagnostische Verfahren, die dieses neuartige und unerwartete Tumorepitop als Zielstruktur verwenden, verbessern bisherige Ansätze, die das MUC1 nach den bisherigen bekannten tumorassoziierten Eigenschaften Überexpression, apolare Expression und aberrante Glykosylierung betrachten, signifikant.

Hierin beschrieben ist ein neuartiges und überraschendes Tumorepitop auf dem humanen polymorphen epithelialen Muzin MUC1. Ferner wird hierin dieses Epitop sowie Substanzen und Verfahren zu deren Herstellung, die dieses Epitop enthalten, wie beispielsweise Zellen, Glykoproteine oder Glykopeptide, und Verfahren zur Anwendung in der Prophylaxe und Behandlung von Tumorerkrankungen, einschließlich Vakzine, wie beispielsweise Ganzzellvakzine, Glykoprotein- oder Glykopeptidvakzine, sowie Antikörper, die mit Hilfe von MUC1 als Immunogen gewonnen wurden, das durch das erfindungsgemäße Herstellungsverfahren gewonnen wurde, und die einen erhöhten additiven Effekt aufweisen und deshalb besonders zur Anwendung in der Behandlung und/oder Diagnostik von Tumorerkrankungen geeignet sind, beschrieben.

Für Anwendungen von MUC1 in Tumortherapie und -prophylaxe ist es notwendig solche MUC1-Moleküle zu isolieren und/oder zu identifizieren, die tumorrelevante Epitope tragen und möglichst keinen oder einen verringerten immunsuppressiven Einfluss haben. Dies gilt auch für die Isolierung und Herstellung von Zellen oder anderen Trägersubstanzen, die geeignete MUC1-Moleküle im Sinne der Erfindung tragen. Für die Diagnostik ist es vorteilhaft, eine differentielle Diagnostik zu erreichen, die bestimmen kann, ob und in weichem Maße MUC1 vorliegt, das tumorrelevante Epitope trägt oder weniger beziehungsweise irrelevante Epitope trägt, sowie nicht, verringert oder vermehrt immunsuppressiv wirkt (siehe auch weiter unten Diagnostik). Die im Folgenden beschriebenen erfindungsgemäßen Verfahren erlauben die Definition, Charakterisierung und Isolierung der obigen erfindungsgemäß geeigneten MUC1-Moleküle.

Die Erfindung betrifft Verfahren, die es erlauben, aus dem großen Pool an unterschiedlichen MUC1-Molekülen, deren Strukturen in keinem Fall detailliert chemisch bekannt sind, solche MUC1-Moleküle oder deren Fragmente oder Derivate zu identifizieren und/ oder zu gewinnen, die für eine Anwendung in der Tumorbehandlung, Tumorprophylaxe und Tumordiagnose vorteilhaft, verwendet werden können. Solche MUC1-Moleküle werden im weiteren erfindungsgemäß als tumorassoziierte MUC1-Moleküle bezeichnet und beinhalten neben Molekülen auch Molekülmischungen, wie beispielsweise Glykopeptide, Glykoproteine oder Mischungen daraus sowie Fragmente und Derivate der Moleküle, die das erfindungsgemäße Tumorepitop enthalten. Erfindungsgemäß sind tumorassoziierte Moleküle auch Zellen oder andere Trägersubstanzen, wie beispielsweise Viren, Bakterien, Teile von Zellen, wie beispielsweise Exosomen oder Zelllysate, oder Liposomen, die ein oder mehrere tumorassoziierte MUC1-Moleküle mit dem erfindungsgemäßen Tumorepitop enthalten. Die Verfahren entsprechen Screening-Assays die es erlauben für die oben genannten und im Folgenden im Detail ausgeführten Anwendungen geeignetes MUC1 zu identifizieren und zu gewinnen. Der zentrale Schritt des Herstellungsverfahren ist eine Identifizierung - und/oder Isolierung von tumorassoziierten MUC1-Molekülen mit Hilfe von Antikörpern, die das erfindungsgemäße MUC1-Tumorepitop erkennen, wie beispielsweise Antikörper der Gruppe 1 (A76-A/C7, VU-11E2, VU-11D1, BC4E549, VU-12E1, VU-3D1 und b-12). Eine bevorzugte Gruppe an Antikörpern sind dabei solche Antikörper, die einen additiven Bindungseffekt aus Länge und PDTR-Glykosylierung aufweisen, wie beispielsweise A76-A/C7, VU-11E2, VU-11D1, BC4E549, VU-12E1, VU-3D1 und b-12. Eine weiter bevorzugte Gruppe an Antikörpern sind hierbei Antikörper mit einem besonders starken additiven Effekt, wie beispielsweise A76-A/C7, VU-11 E2 und VU-11D1. Erfindungsgemäß ist der Antikörper A76-A/C7 für alle Anwendung zu bevorzugen, da er den stärksten additiven Effekt aufweist. Ein weiterer Antikörper, der für das erfindungsgemäße Herstellungsverfahren geeignet wäre, ist der VU-4H5, der ebenfalls einen starken additiven Effekt aufweist, ansonsten aber invers glykosylierungsabhängig bezüglich kurzer PDTR-glykosylierter Tandem Repeats ist (siehe Beispiel 4). In diese Gruppe fallen auch andere Antikörper mit vergleichbaren Bindungseigenschaften.

Die in den Verfahren verwendeten einzelnen Methoden sind dem Fachmann bekannt und in den Beispielen beispielhaft im Detail erläutert.

Tumorassoziierte MUC1-Moleküle werden in einem erfindungsgemäßen Verfahren durch Bindung an die oben beschriebenen Antikörper, identifiziert und/ oder isoliert und gewonnen.

Die Methoden die für die Identifizierung verwendet werden sind an sich bekannte Methoden, beispielweise ELISA, RIA, Westernblot, Immunpräzipitation, Scatchard-Blot-Analysen, FACS-Analysen, MACS-Analsysen, Immuncytochemie und Immunhistologie. Die meisten dieser Methoden sind im Detail in den Beispielen beschrieben.

Nach dem erfindungsgemäßen Verfahren können die oben beschriebenen MUC1-Moleküle aus Überständen von Zellkulturen von ZR-75-1 Zellen durch eine Affinitätschromatographie an einem oder mehreren Antikörpern wie oben gezeigt gewonnen werden. Dabei können weitere Reinigungs- und/ oder Konzentrierungsschritte nach an sich bekannten Methoden mit einem oder mehreren Affinitätschromatographieschritten an den Antikörpern kombiniert werden. Ebenso könnten tumorassoziierte MUC1-Moleküle aus Tumorzellen, Tumorgeweben oder Tumorzelllinien gewonnen werden, indem ein geeigneter Schritt nach an sich bekannten Methoden vorgeschaltet wird, der es erlaubt das auf den Zellen membrangebundene oder das intrazelluläre MUC1 für die Affinitätsreinigung zugänglich zu machen, beispielsweise durch Solubilisierung mit geeigneten Detergenzien oder durch Abspaltung extrazellulärer MUC1-Bestandteile durch Proteolyse oder durch Zelllyse. Eine differenzielle Reinigung der tumorassoziierten MUC1-Moleküle aus Überständen von Zellkulturen und aus Körperflüssigkeiten getrennt von membrangebundenem tumorassoziierten MUC1 aus Tumorzellen, Tumorgeweben oder Tumorzelllinien wird durch eine Kombination der oben beschriebenen Methoden ermöglicht. Ein wichtiger Schritt hierbei ist die Trennung von Überständen und Zellen nach an sich bekannten Methoden beispielsweise durch Zentrifugation. Für eine Herstellung von sezernierten und membranständigen tumorassoziierten MUC1-Molekülen wird eine Kombination beider Verfahren verwendet, beispielsweise durch Vereinigung der Zellkulturüberstände mit solubilisierten membranständigem MUC1 vor entsprechenden Affinitätschromatographieschritten an geeigneten Antikörpern. Erfindungsgemäße tumorassoziierte MUC1-Moleküle sind auch solche, die durch weitere Affinitätschromatographieschritte mit anderen tumorspezifischen Antikörpern, beispielsweise Antikörper oder Lektine gegen tumorassoziierte oder tumorspezifische Kohlenhydrat Antigene, beispielsweise das Thomsen-Friedenreich Antigen, das Tn-Antigen, das Sialyl-Tn-Antigen oder das Lewis Y-Antigen, weiter gereinigt oder vorgereinigt wurden, um tumorassoziierte MUC1-Moleküle zu erzeugen, die multiple Tumorantigene tragen. Solche tumorassoziierten MUC1-Molekiile sind für die Tumortherapie von Vorteil, da Immunantworten, die durch sie gegen den Tumor erzeugt werden, für Tumorescape-Mechanismen schwieriger zu umgehen sind.

Fragmente von tumorassoziierten MUC1-Molekülen können nach an sich bekannten Methoden vor oder nach Affinitätschromatographie an geeigneten Antikörpern, beispielsweise durch enzymatische oder chemische Proteolyse, hergestellt und durch geeignete an sich bekannte Methoden gereinigt werden. Einer Fragmentierung kann dabei eine partielle Deglykosylierung vorgeschaltet sein, beispielsweise durch ein oder mehrere enzymatische Deglyksoylierungsschritte, wie beispielsweise eine komplette oder partielle Abspaltung von Sialinsäuren durch Sialidasen, oder eine komplette oder partielle Abspaltung von Fucose-Bausteinen durch ein oder mehrere chemische Deglykosylierungsschritte, wie beispielsweise nach der an sich bekannten TFMSA-Methode (Trifluormethylsulfonsäure Behandlung).

Tumorassoziierte MUC1-Moleküle und deren Fragmente können weiter nach an sich bekannten Methoden modifiziert werden. Zu erfindungsgemäßen Modifikationen zählen dabei beispielsweise die Änderungen der Glykosylierung in ein oder mehreren Schritten, beispielsweise die enzymatische Glyksoylierung oder Deglykosylierung mit Glykosyltransferasen und/ oder Glyksoylidasen und/ oder chemischer Deglykosylierungen. Die Modifikationen können vor oder nach Affinitätschromatographieschritten an Antikörpern erfolgen. Die resultierenden Fragmente können durch geeignete Reinigungs- und/ oder Konzentrierungsschritte nach an sich bekannten Methoden weiter gereinigt beziehungsweise fraktioniert werden. Weitere erfindungsgemäße Modifikationen sind Kopplungen mit anderen Substanzen, beispielsweise solche, die eine Immunantwort gegen MUC1 auf Tumorzellen erhöhen und/ oder eine Applikation zur Behandlung und/ oder Prophylaxe von Tumoren und /oder Metastasen im Menschen begünstigen, beispielsweise Kopplung der tumorassoziierten MUC1-Moleküle an Trägermoleküle, beispielsweise KLH, oder an immunstimulatorische Moleküle, beispielsweise Interleukine, GM-CSF, Interferone und / oder TNF-alpha, oder solche, die die Stabilität der Moleküle in vivo und während der Lagerung erhöhen, oder solche, die eine Expression erhöhen, oder solche die andere Produktionstechnische Vorteile bewirken, wie beispielsweise Löslichkeit. Die Kopplungen erfolgen nach an sich bekannten Methoden beispielsweise chemisch über geeignete Spacer oder biochemisch in Form von Fusionsproteinen, die rekombinant in Zellen exprimiert werden (siehe weiter unten), beispielsweise Interleukin 12 oder Tetanus Toxoid-Peptid.

Ein Verfahren zur Herstellung tumorassoziierter MUC1-Moleküle ist auch die Synthese von Glykopeptiden nach an sich bekannten Methoden, die von den beschriebenen Antikörpern, die das MUC1- Tumorepitop erkennen, besser gebunden werden als die identischen Glykopetide, die jedoch nicht an der PDTR-Region glykosyliert sind. Vorteilhaft kann dabei sein, tumorassoziierte MUC1-Moleküle, die durch eine der oben beschriebenen Verfahren gewonnen wurden, ganz oder in Teilen zu synthetisieren und durch ein oder mehrere zusätzliche Aminosäuren, beispielsweise Cystein oder Lysin and Trägermoleküle, mit an sich bekannten Molekülen zu koppeln, beispielsweise KLH. Eine komplette oder partielle Analyse erfolgt durch an sich bekannte Methoden, beispielsweise der fragmentierenden Massenspektrometrieanalyse, beispielsweise PSD-MALDI-MS oder ESI-MS/MS, oder NMR oder immunchemischen Methoden.

MUC1-Glykopeptide werden dadurch gewonnen, daß Glykopeptide nach an sich bekannten Methoden synthetisiert werden und ihre Bindung an MUC1-Antikörper getestet wird. MUC1-Glykopeptide, die durch Antikörper gebunden werden, die das hierin beschriebene MUC1-Tumorepitop erkennen, und diese besser binden als die identischen Glykopetide, die jedoch nicht an der PDTR-Region glykosyliert sind, entsprechen den erfindungsgemäßen Kriterien für MUC1-Glykopeptide (die erwähnten Bindungseigenschaften dieser Antikörper sind bereits weiter oben unter Punkt ii) ausgeführt worden. Bevorzugte MUC1-Glykopeptide sind solche, die durch Antikörper besser gebunden werden, als entsprechende kürzere Glykopeptide mit 1 oder 2 Tandem Repeats aber gleicher Glykosylierung and gleichen Glykosylierungsstellen (die erwähnten Bindungseigenschaften dieser Antikörper sind bereits weiter oben unter Punkt iv) ausgeführt worden. In einer bevorzugten Variante wird die Peptid-Chip-Technologie verwendet. Hierbei wird eine Großzahl von MUC1-Peptiden hergestellt, die an unterschiedlichen Positionen glykosyliert sind und/oder unterschiedliche Länge. Mit Hilfe der Bindung von Antikörpern gegen das hierin beschriebene MUC1-Tumorepitop werden solche MUC1-Glykopeptide in dem HT-Verfahren (high throughput) gewonnen, die den erfindungsgemäßen Kriterien für MUC1-Glykopeptide entsprechen. In einer weiter bevorzugten Variante können mit der gleichen Methode solche MUC1-Glykopeptide gewonnen werden, die darüber hinaus besonders immunogen für Kohlenhydratepitope sind, indem die oben beschriebenen Peptid-Chips zusätzlich mit tumor-spezifischen anti-Kohlenhydrat-Antikörpern getestet werden und solche Glykopeptide identifiziert werden, die von Antikörpern gegen das erfindungsgemäße MUC1-Tumorepitop und von Antikörpern gegen das tumorspezifische Kohlenhydratantigene gebunden werden. Die HT-Verfahren sind für Peptide im Prinzip bekannt und können auf Glykopeptide übertragen werden. Verfahren zur Herstellung von Strukturen, die mehrere bis viele tumorassoziierte MUC1-Moleküle tragen, erfolgen nach an sich bekannten Verfahren, beispielsweise durch chemische Kopplung an Liposomen oder Kopplung an Lipide und nachfolgende Einlagerung in Liposomen oder durch Kopplung an andere Trägerstrukturen.
Wie nachfolgend noch ausgeführt wird, könnten auch Zellen oder Zelllinien, die MUC1-Moleküle oder deren Fragmente tragen oder sezernieren oder sezernieren und tragen, die durch die oben beschriebenen Verfahren hergestellt werden können, in den erfindungsgemäßen Verfahren eingesetzt werden. Verfahren zur Herstellung solcher Zellen beinhalten die Selektion von Zellen, die die tumorassoziierten MUC1-Moleküle tragen durch Bindung an Antikörper, die das Tumorepitop erkennen (Gruppe 1, bevorzugt A76-A/C7). Hierfür werden an sich bekannte Methoden verwendet. Beispielsweise werden die Zellen mit tumorassoziierten MUC1-Molekülen durch Magnettrennung mit Hilfe an Magnet bindende, mit den Antikörpern beladene Partikel gewonnen (MACS-Sortierung) und von solchen Zellen, die keine oder geringe Mengen an tumorassoziierten MUC1-Molekülen tragen, getrennt. Ein weiteres Beispiel ist die Gewinnung - mit Hilfe eines FACS-Sorters - der Zellen, die die Antikörper tragen, die fluoreszenzmarkiert wurden, sortiert. Beide Methoden sind dem Fachmann bekannt. Dabei ist es vorteilhaft, die durch die Antikörperbindung gewonnenen Zellen durch Vereinzelung (limitierende Verdünnnungen) in geeigneten Medien nach an sich bekannten Methoden zu klonieren, um stabile Zellkione zu gewinnen und über Antikörperbindung, beispielsweise mit Hilfe von quantitativen ELISA, nach an sich bekannten Methoden solche Zellklone mit Hilfe der Antikörper zu identifizieren und auszuwählen, die eine geeignete Menge an tumorassoziiertem MUC1 sezernieren, tragen oder tragen und sezernieren (siehe Beispiel 5. Ein Teil des Herstellungsverfahren ist dabei in bevorzugter Weise ein quantitativer Sandwich-ELISA, der weiter unten im Detail erläutert wird (Testverfahren quantitativer Sandwich-ELISA, siehe Beispiel 5). Eine alternative Methode, die auch ergänzend zu verwenden ist, ist eine quantitative Bestimmung der MUC1-positiven Zellen und der Menge an tumorassoziierten MUC1-Molekülen auf den Zellen über eine FACS-Bestimmung oder einer Scatchard-Blot-Analyse, die an sich dem Fachmann bekannt ist, mit Hilfe der hierin beschriebenen Antikörper, die das MUC1-Tumorantigen erkennen. Die Zellen oder Zelllinien könnten entweder selbst als tumorassoziierte MUC1-Moleküle verwendet werden oder es werden aus den Zellen nach oben beschriebenen Verfahren sezernierte oder membranständige oder sezernierte und membrabständige Glykoproteine, Glykopeptide, oder deren Fragmente gewonnen. Auch Zelllysate werden nach an sich bekannten Methoden, beispielsweise durch abwechselndes Frieren mit flüssigem Stickstoff und Tauen hergestellt.

Ein weiteres Verfahren zur Herstellung von Zellen oder Zelllinien enthält zusätzlich zu den oben beschriebenen Verfahren eine Transformation von Zellen mit rekombinantem MUC1. Hierzu werden geeignete Zellen oder Zelllinien ausgewählt und entweder die Expression des MUC1 erhöht, neue Formen, beispielsweise Fragmente, des MUC1 exprimiert oder MUC1 de novo exprimiert mit dem Ziel tumorassoziierte MUC1-Moleküle zu bekommen, beziehungsweise Zellen zu gewinnen, die mehr oder verbesserte tumorassoziierte MUC1-Moleküle tragen oder sezernieren. Hierzu wird das MUC1-Gen oder Teile davon verwendet. Bevorzugt umfasst das MUC1-Gen eine DNA-Sequenz ausgewählt aus der Gruppe von bei NCBI hinterlegten Sequenzen bestehend aus: Hinterfegungsnummer NM 002456, XM 053256, AF125525, AF348143, S81781, X80761, X69118, J05582 und M21868. Bevorzugt sind solche Teile des MUC1-Gens, die verkürzte Versionen, die ein oder mehrere Tandem Repeats enthalten, codieren in einen geeigneten Expressionsvektor nach an sich bekannten Methoden kloniert und in die Zellen eingebracht, beispielsweise durch Elektroporation. Selektion der transformierten Zellen erfolgt nach an sich bekannten Methoden beispielsweise über Selektion mit geeigneten Antibiotika deren Resistenzen auf dem rekombinant eingebrachten genetischen Material mit codiert ist (siehe Beispiel 5). Stabil transformierte Zellen werden bevorzugt, wie oben beschrieben, kloniert, beispielsweise durch limitierende Verdünnung vorzugsweise in Verbindung mit einer Anreicherung der tumorassoziierten MUC1-Moleküle exprimierenden Zellen beispielsweise durch FACS oder MACS (siehe Beispiel 5).
In einem weiteren Verfahren können tumorassoziierte MUC1-Molekül positive Zellen oder Zelllinien durch Gentransfer sensitiv für die Behandlung von Zytostatika gemacht werden, beispielsweise durch die Einführung des HSV-TK Gens, das dazu führt, dass die Zellen sensitiv für eine Gancyclovir-Behandlung werden. Das Einbringen der Gene und die Selektion transformierter Zellen erfolgt nach an sich bekannten Methoden. Die Gancyclovir-sensitiven Zellen werden erfindungsgemäß entweder vor der Anwendung am Menschen oder nach Applikation am Menschen durch Gancyclovir abgetötet. Dieses Verfahren erlaubt die Kontrolle von im Menschen applizierten lebenden Zellen oder Zelllinien in der Tumorbehandlung und wird alternativ oder ergänzend zur Bestrahlung von Zellen zu diesem Zweck angewendet (siehe auch erfindungsgemäße Verwendungen).
In einem weiteren Verfahren können tumorassoziierte MUC1-Moleküle aus Geweben gewonnen werden, indem das tumorassoziierte MUC1-Molekül mit Hilfe der weiter oben beschrieben Techniken aus den Membranen der Zellen gewonnen wird. Hierzu wird das Gewebe nach an sich bekannten Methoden aufgeschlossen, um die membranständigen tumorassoziierten MUC1-Moleküle zugänglich zu machen, beispielsweise durch proteolytische oder mechanische Methoden.

Die Verfahren werden in den Beispielen im Detail weiter erläutert sind jedoch nicht auf diese beschränkt.

Mit Hilfe der Herstellungsverfahren können tumorassoziierte MUC1-Moleküle gewonnen werden, die nicht oder vermindert immunsuppressiv sind, und eine Immunantwort gegen das native Tumor-MUC1 und gegen Tumorzellen erzeugen. Im Beispiel 5 wird gezeigt, wie erfindungsgemäße tumorassoziierte MUC1-Moleküle nicht oder vermindert immunsuppressiv wirken. Solche erfindungsgemäße tumorassoziierte MUC1-Moleküle sind immunogen und können eine Anti-Tumor-Immunantwort erzeugen.
Ein Teil des Herstellungsverfahren ist dabei bevorzugterweise ein quantitativer Sandwich-ELISA, bei dem ein anti-MUC1-Antikörper immobilisiert wird, Zellen oder gewonnenes MUC1 aus Überständen oder Membranpräparationen in geeigneten Verdünnungen aufgetragen und durch einen zweiten anti-MUC1-Antikörper nachgewiesen wird. Mindestens ein Antikörper ist dabei gegen das hierin beschriebene tumorassoziierte MUC1-Molekül gerichtet. Der zweite Antikörper ist bevorzugt gegen ein Epitop auf MUC1 gerichtet, das nicht in der PDTR-Region liegt oder gegen ein Kohlenhydratepitop, beispielsweise Thomsen-Friedenreich Antigen. Dieser quantitative Sandwich-ELISA ist ein
Testverfahren für die Herstellung von tumorassoziierten MUC1-Molekülen, einschließlich von Zellen, sowie ein Testverfahren für die Diagnostik.
Antikörper gegen MUC1 werden zum Teil bereits in der Diagnostik eingesetzt und zur Behandlung von Tumoren in der Klinik erprobt. Allerdings erkennen die MUC1-spezifischen Antikörper unterschiedliche Epitope auf MUC1, das eine Vielzahl unterschiedlicher Epitope aufweist und außerdem, wie oben beschrieben, kein genau strukturell definiertes Molekül sondern eine heterogene Mischung von Molekülen ist, das sich vom Tumor zum Normalgewebe unterscheidet, beispielsweise in der Glykosylierung. So wird zur Zeit beispielsweise der HMFG-1 in der Klinik geprüft, der nicht das hierin beschriebene MUC1-Tumorepitop erkennt.
Hierin ist gezeigt, welche Antikörper aufgrund der Bindungseigenschaften für die Behandlung von Tumoren von Vorteil sind. Damit wird eine Auswahl von besser geeigneten Antikörpern für die Tumorbehandlung in Form eines Screening-Verfahrens (Screening-Assay) ermöglicht. Weiterhin ist ein Verfahren zur Bestimmung der Eignung von Antikörpern für die Behandlung von Tumoren und zur Diagnostik und zur Erkennung des Tumorepitops beschrieben. Dieses Verfahren umfasst eine Kombination von biochemischen Tests, die in den Beispielen 1 und 2 und bevorzugt auch 3 beschrieben sind. Antikörper, die die definierten Bindungseigenschaften der Gruppe 1 oder des Beispiels 4 in dem Verfahren aufweisen, sind im Sinne der Erfindung geeignet, wie zuvor beschrieben wurde. Mit Hilfe dieses Verfahrens werden auch Antikörper für die Eignung zur Bestimmung des hierin beschriebenen MUC1-Tumorepitops und zur Charakterisierung, Isolierung und Gewinnung von tumorassoziierten MUC1-Molekülen charakterisiert und gewonnen.
Weiterhin ist hierin ein Verfahren zur Herstellung von besonders geeigneten monoklonalen Antikörpern zur Therapie und Diagnose von Tumorerkrankungen beschrieben, da diese im Vergleich zu bisher getesteten Antikörpern einen besonders starken additiven Effekt aufweisen. Dabei wird MUC1-Material in Form von Molekülen, das mit den erfindungsgemäßen Verfahren gewonnen wurde, in einem Verfahren zur Immunisierung von Tieren oder Menschen verwendet. Die Herstellung und Charakterisierung der Antikörper erfolgt dabei nach an sich bekannten Methoden unter Einschluß von mindestens einem der oben beschriebenen Testsysteme. Die Spezifität der Antikörper wird dabei auf besonders hohe additive Effekte getestet.

Vorteilhafterweise ermöglichen die erfindungsgemäßen Verfahren die Herstellung oder Identifizierung von tumorassoziierten MUC1-Molekülen, die in der Lage sind im Menschen eine stimulatorische Immunantwort hervorzurufen, und somit eine geeignete Ausgangssubstanz für Impfstoffe gegen Tumore darstellen, die tumorassoziiertes MUC-1 produzieren. Die Herstellung dieser MUC1-Moleküle wird erst durch die erfindungsgemäßen Verfahren ermöglicht. Die Erfindung beruht, inter alia, auf der überraschenden Beobachtung, dass in Mäusen hergestellte Antikörper gegen tumorassoziiertes MUC1, die die oben beschriebenen Eigenschaften aufweisen, in der Lage sind, in der heterogenen Gruppe an tumorassoziierten MUC1-Molekülen solche spezifisch zu binden, die im Menschen in der Lage sind, eine Immunantwort auszulösen, wie beispielsweise mit Hilfe von NOD-SCID-Maus-Versuchen gezeigt werden kann. Vorzugsweise zeichnen sich diese MUC1-Moleküle dadurch aus, dass sie eine erhöhte Anzahl an immunstimulatorisch wirkenden Epitopen und eine verringerte Anzahl oder gar keine immunsuppressiven Epitope aufweisen.

Die zuvor eingeführten Definitionen der Begriffe sind für die Begriffe in den nachfolgend beschriebenen Verfahren mutatis mutandis anwendbar.

Weiterhin ist hierin ein Verfahren zur Identifizierung eines MUC1-Moleküls beschrieben, welches in der Lage ist, eine Immunantwort im Menschen hervorzurufen, umfassend:

a) in Kontakt bringen eines Gemisches von MUC1-Molekülen mit einem Antikörper welcher die folgenden Eigenschaften aufweist:

i) Bindung an die immundominante Region des MUC1-Tandem Repeats; und
ii) Bindung an ein MUC1-Fragment, welches zwischen 9 und 40 Aminosäuren lang ist und Sequenzen des MUC1-Tandem Repeats und der immundominanten Region enthält, wird durch Glykosylierung des Threonins einer PDTR-Sequenz ermöglicht oder erhöht; und
iii) Bindung an nicht-glykosylierte MUC1-Tandem Repeats wird erhöht, wenn die nicht glykosylierten MUC1-Tandem Repeats in Serie vorliegen; und
iv) Bindung, an multiple glykosylierte MUC1-Tandem Repeats, die Glykosylierungen an den Threoninen mehrerer PDTR-Sequenzen der immundominanten Region tragen, ist gemäß eines addditiven Effektes aus Länge und PDTR-Glykosylierung gegenüber kurzen MUC1-Fragmenten aus ii) erhöht
für einen Zeitraum der ausreichend ist und unter Bedingungen die geeignet sind, damit sich ein Immunkomplex ausbildet; und

b) Identifizierung des Immunkomplexes.

Unter dem Begriff "Identifizieren" sind alle Maßnahmen zu verstehen, durch die ein Immunkomplex nachgewiesen werden kann. Geeignete Methoden sind im Stand der Technik bekannt und umfassen beispielsweise ELISA, MACS, RIA, Scatchard-Blot-Analyse, Westernblot, Immuncytochemie, Immunhistologie oder FACS Verfahren. Geeignete Verfahren werden auch nachfolgend noch genauer beschrieben.
In einer bevorzugten Ausführungsform umfassen die erfindungsgemäßen Verfahren, weiterhin mindestens einen der folgenden Schritte:

1) Gewinnung des MUC1-Moleküls oder des Gemisches davon in Schritt (a) des erfindungsgemäßen Verfahrens aus Tumorgewebe, Tumorzellen und/oder Körperflüssigkeiten die tumorassoziierte MUC1-Moleküle enthalten, welche(s) zuvor isoliert wurde(n);

2) Gewinnung des MUC-1-Moleküls oder des Gemisches davon in Schritt (a) des erfindungsgemäßen Verfahrens Zellen oder Zelllinien, die tumorassoziierte MUC1-Moleküle oder Gemische davon exprimieren und/oder sezernieren;

3) Gewinnung des MUC1-Moleküls oder des Gemisches davon in Schritt (a) des erfindungsgemäßen Verfahrens aus rekombinanten Zellen oder Zelllinien, die zuvor gentechnisch so verändert wurden, dass sie tumorassoziierte MUC1-Moleküle oder Gemische davon exprimieren und/oder sezernieren;

4) Gewinnung des MUC1-Moleküls oder des Gemisches davon in Schritt (a) des erfindungsgemäßen Verfahrens aus Zelllysaten und/oder dem Zellüberstand von Tumorgeweben, Tumorzellen und/oder Körperflüssigkeiten wie unter (1) beschrieben oder aus Zellen oder Zelllinien wie unter (2) und (3) beschrieben, die tumorassoziiertes MUC1 enthalten.

Geeignete Tumorgewebe oder Tumorzellen können aus Tumorpatienten oder Personen ohne Tumor gewonnen werden, wobei im letzten Fall eine weitere Behandlung, wie andernorts beschrieben, durch beispielsweise partielle Deglykosylierung vor der Affinitätsreiniung notwendig sein kann.
Eine bevorzugte Form sind tumorassoziierte MUC1-Moleküle in der Form von Glykoproteinen und Glykopeptiden, die aus dem Überstand von Zellen gewonnen werden, die tumorassoziierte MUC1-Moleküle in großer Menge sezernieren. In dem bevorzugten Verfahren wurde eine Subzellinie der Mammakarzinom Zelllinie ZR-75-1 mit Hilfe von ELISA Techniken, FACS-Analysen und immunzytochemischen Tests als eine geeignete Zellline identifiziert und charakterisiert, die tumorassoziierte -MUC1-Moleküle auf der Oberfläche exprimieren und in den Zellkulturüberstand abgeben (sezernieren), siehe auch Beispiel 5. Da die Expressionsrate der einzelnen Zellen unterschiedlich ist, wurde aus der Ausgangszelllinie durch limitierende Verdünnung Klone (Sublinien) hergestellt, die stabil erhöhte Mengen an tumorassoziierten MUC1-Molekülen tragen und/ oder sezernieren, siehe auch Beispiel 5. In einem weiteren bevorzugten Verfahrenschritt wurde, um die Expression der sekretorischen tumorassoziierten MUC1-Moleküle weiter zu erhöhen, das MUC1-Gen oder Teile davon in einem im Beispiel 5 näher beschriebenen Expressionsvektor und Verfahren in die Zelle integriert. Die Zellen wurden wieder durch limitierende Verdünnungen in geeigneten Kulturmedien kloniert. Aus den entstandenen Sublinien wird aus dem Überstand mit Hilfe einer Affinitätschromatographie an A76-A/C7 tumorassoziierte MUC1-Moleküle gewonnen, die in Verfahren in der Tumorbehandlung als Tumormittel und in der Tumordiagnostik eingesetzt werden.
In einem weiteren bevorzugten Verfahren werden aus den gewonnenen tumorassoziierten Molekülen, durch limitierte Proteolyse mit Clostripain nach an sich bekannten Methoden Fragmente hergestellt.
In einem weiteren bevorzugten Verfahren werden durch an sich bekannte Methoden tumorassoziierte MUC1-Glykopeptide synthetisch hergestellt. Dabei handelt es sich um MUC1-Glykopeptide, die durch Antikörper gebunden werden, die das hierin beschriebene MUC1-Tumorepitop erkennen, und diese besser binden als die identischen Glykopetide, die jedoch nicht an der PDTR-Region glykosyliert sind. Bevorzugte erfindungsgemäße MUC1-Glykopeptide sind solche, die durch Antikörper besser gebunden werden, als entsprechende kürzere Glykopeptide mit 1 oder 2 Tandem Repeats aber gleicher Glykosylierung and gleichen Glykosylierungsstellen. Die entsprechenden Bindeeigenschaften wurden bereits weiter oben ausgeführt.
Das erfindungsgemäße Verfahren zur Anwendung am Menschen beinhaltet geeignete pharmakologische Formulierungen, bevorzugt in Kombination mit Adjuvantien und/ oder costimulatorischen Molekülen, wie beispielsweise GM-CSF und bei kürzeren MUC1-Glykopeptiden eine Kopplung an KLH. Eine hierbei bevorzugte Variante ist die Beladung von dendritischen Zellen oder Zellen, die eine immunstimulatorische Funktion vergleichbar zu dendritischen Zellen besitzen, mit Zelllysaten oder die Fusion von Zellen, die das tumorassoziierte MUC1-Molekül tragen mit dendritischen Zellen nach an sich bekannten Verfahren und die Verabreichung von diesen Zellen, bevorzugt nach Bestrahlung, am Menschen nach an sich bekannten Methoden. Die Behandlung mit beladenen dendritischen Zellen und Glykopeptiden und/ oder Glykoproteinen wird vorzugsweise kombiniert, wobei spätere Boosterungen bevorzugt mit den erfindungsgemäßen Glykopeptiden und / oder Glykoproteinen in geeigneten erfindungsgemäßen pharmakologischen Formulierungen, bevorzugt mit Adjuvantien oder costimulatorischen Molekülen (beispielsweise GM-CSF) durchgeführt werden.
Die bevorzugte erfindungsgemäße Verwendung der Glykopeptide und Glykoproteine oder mit Glykopeptiden und/ oder Glykoproteinen beladene dendritische Zellen ist zur Behandlung und Prophylaxe von MUC1-positiven Tumorerkrankungen, wie beispielsweise Mammakarzinome, Ovarialkarzinome, Kolonkarzinome, Gastrointestinalkarzinome, Pankreaskarzinome, Lungenkarzinom, multiples Myelom, und dabei gegen Primärtumoren, minimal residuale Tumorerkrankungen" Metastasen und als adjuvante Behandlung. Bevorzugterweise werden die Glykopeptide und Glykoproteine oder mit Glykopeptiden und/ oder Glykoproteinen beladene dendritische Zellen nach an sich bekannten Methoden subkutan, intradermal, intrarectal, intranodal (Lymphknoten) oder systemisch verabreicht.

Weiterhin ist hierin ein Verfahren zur Herstellung von Zellen beschrieben, die ein MUC1-Molekül umfassen, welches in der Lage ist eine Immunantwort im Menschen hervorzurufen, umfassend:

a) in Kontakt bringen eines Gemisches von Zellen, die MUC1-Moleküle umfassen, mit einem Antikörper welcher die folgenden Eigenschaften aufweist:

    i) Bindung an die immundominante Region des MUC1-Tandem Repeats; und
    ii) Bindung an ein MUC1-Fragment, welches zwischen 9 und 40 Aminosäuren lang ist und Sequenzen des MUC1 Tandem Repeats und der immundominanten Region enthält, wird durch Glykosylierung des Threonins einer PDTR-Sequenz ermöglicht oder erhöht; und
    iii) Bindung an nicht-glykosylierte MUC1-Tandem Repeats wird erhöht, wenn die nicht glykosylierten MUC1-Tandem Repeats in Serie vorliegen; und
    iv) Bindung, an multiple glykosylierte MUC1-Tandem Repeats, die Glykosylierungen an den Threoninen mehrerer PDTR-Sequenzen der immundominanten Region tragen, ist gemäß eines addditiven Effektes aus Länge und PDTR-Glykosylierung gegenüber kurzen MUC1-Fragmenten aus ii) erhöht für einen Zeitraum der ausreichend ist und unter Bedingungen die geeignet sind, damit sich ein Immunkomplex ausbildet;

b) Isolierung der Zellen, die einen Immunkomplexes gebildet haben; und
c) Bereitstellung der Zellen aus dem Immunkomplex.

Der Begriff "Zellen, die ein MUC1-Mofekül umfasst, welches in der Lage ist eine Immunantwort im Menschen hervorzurufen", umfasst alle Zellen, die ein tumorassoziiertes MUC1-Molekül wie oben beschrieben produzieren. Solche Zellen sind vorzugsweise Zellen, die aus Tumorgewebe isoliert oder hergestellt werden können, Tumorzelllinien, oder Zellen oder Zelllinien, die gentechnisch so verändert wurden, dass sie ein tumorassoziiertes MUC1-Molekül wie oben beschrieben produzieren. Die zuletzt genannten Zellen oder Zelllinien können durch im Stand der Technik bekannte Methoden hergestellt werden. Dies sind z.B. Verfahren, mit denen Fremd- DNA stabil oder transient in die Zellen oder Zelllinien eingebracht werden kann. Weitere geeignete Verfahren werden auch nachfolgend genauer beschrieben.

Unter dem Begriff "Isolierung der Zellen" sind alle Maßnahmen zur Separierung von Zellen zu verstehen, die durch die von ihnen produzierten tumorassoziierten MUC1-Moleküle einen Immunkomplex mit Antikörpern gebildet haben. Dem Fachmann sind diese Verfahren bekannt. Vorzugsweise wird hierfür die FACS- oder die MACS-Methode eingesetzt. Weitere geeignete Verfahren sind nachfolgend genauer beschrieben.

Die zuvor eingeführten Definitionen der Begriffe sind für die Begriffe in den nachfolgend beschriebenen Verfahren mutatis mutandis anwendbar.

Weiterhin ist hierin ein Verfahren zur Identifizierung von Zellen beschrieben, die ein MUC-1-Molekül umfassen, welches in der Lage ist eine Immunantwort im Menschen hervorzurufen, umfassend:

a) in Kontakt bringen eines Gemisches von Zellen, die MUC1-Moleküle umfassen, mit einem Antikörper, welcher die folgenden Eigenschaften aufweist:

    i) Bindung an die immundominante Region des MUC1-Tandem Repeats; und
    ii) Bindung an ein MUC1-Fragment, welches zwischen 9 und 40 Aminosäuren lang ist und Sequenzen des MUC1-Tandem Repeats und der immundominanten Region enthält, wird durch Glykosylierung des Threonins einer PDTR-Sequenz ermöglicht oder erhöht; und

iii) Bindung an nicht-glykosylierte MUC1-Tandem Repeats wird erhöht, wenn die nicht glykosylierten MUC1-Tandem Repeats in Serie vorliegen; und

iv) Bindung, an multiple glykosylierte MUC1-Tandem Repeats, die Glykosylierungen an den Threoninen mehrerer PDTR-Sequenzen der immundominanten Region tragen, ist gemäß eines addditiven Effektes aus Länge und PDTR-Glykosylierung gegenüber kurzen MUC- Fragmenten aus ii) erhöht

für einen Zeitraum der ausreichend ist und unter Bedingungen die geeignet sind, damit sich ein Immunkomplex ausbildet; und

b) Identifizierung des Immunkomplexes.

Bevorzugt umfassen die hierin beschriebenen Verfahren weiterhin mindestens einen der folgenden Schritte:

1) Gewinnung von Zellen, Zelllininien oder Subzelllinien, die tumorassoziierte MUC1-Moleküle tragen und/oder sezernieren, oder Gemische davon in Schritt (a) des erfindungsgemäßen Verfahrens aus Tumorgewebe oder Tumorzellen, die tumorassoziierte MUC1-Moleküle enthalten, welche zuvor isoliert wurden, mit oder ohne anschließender Zellklonierung;

2) Gewinnung von Zellen, Zelllininien oder Subzelllinien, die tumorassoziierte MUC1-Moleküle tragen und/oder sezernieren, oder Gemische davon in Schritt (a) des erfindungsgemäßen Verfahrens aus Zellen oder Zelllinien die tumorassoziierte MUC1-Moleküle enthalten, mit oder ohne anschließender Zellklonierung;

3) Gewinnung von Zellen, Zelllininien , oder Subzelllinien, die tumorassoziierte MUC1-Moleküle tragen und/oder sezernieren, oder Gemische davon in Schritt (a) des erfindungsgemäßen Verfahrens aus rekombinanten Zellen oder Zelllinien, die zuvor gentechnisch so verändert wurden, dass sie tumorassoziierte MUC1-Moleküle oder Gemische davon exprimieren und/oder sezernieren, mit oder ohne anschließender Zellklonierung;

4) Gewinnung von Zellen, Zelllininien oder Subzelllinien, die tumorassoziierte MUC1-Moleküle tragen und/oder sezernieren, oder Gemische davon in Schritt (a) des erfindungsgemäßen Verfahrens, die gentechnisch so verändert wurden, dass sie immunstimulatorische Moleküle tragen und/oder sezernieren, mit oder ohne anschließender Zellklonierung);

5) Gewinnung von Zelllysaten oder Gemische von Zelllysaten aus Zellen" Zelllinien oder Subzelllinien wie unter (1) bis (4) beschrieben, die tumorassoziiertes MUC1 enthalten.

Besonders bevorzugt ist der Antikörper A76-A/C7 und rekombinante Formen davon, wie nachfolgend noch genauer beschrieben wird.

Der Antikörper A76-A/C7 erkennt das MUC1-Tumorepitop und weist einen besonders starken additiven Effekt auf. Gegenüber dem in der klinischen Entwicklung befindlichen HMFG-1 weißt der A76-A/C7 erfindungsgemäß eine Reihe von Vorteilen auf: Der A76-A/C7 erkennt das erfindungsgemäße MUC1-Tumorepitop und wurde mit Hilfe einer desialinierten Brustkrebszelllinie (T47D) als Immunogen hergestellt. Demgegenüber erkennt HMFG-1 nicht das erfindungsgemäße MUC1- Tumorepitop und wurde mit Hilfe von MUC1 aus humaner Milch als Immunogen hergestellt (siehe auch Beispiele 1 bis 3). A76-A/C7 unterscheidet beispielsweise klar normales Gewebe und gutartige Adenome (negativ) von bösartigen Kolontumoren wie Karzinomen in-situ, Karzinomen und Metastasen (stark positiv), wogegen HMFG-1 diese klare Unterscheidung nicht aufweist. A76-A/C7 ist ein guter Serummarker für die Tumordiagnose, während HMFG-1 nicht in dem Maß als Serum-Tumormarker geeignet ist. Außerdem ist die Affinität des A76-A/C7 um ein Vielfaches höher als HMFG-1. Daher ist der A76-A/C7 im Vergleich zu dem in der klinischen Entwicklung befindlichen HMFG-1 eine deutliche Verbesserung für erfindungsgemäße Anwendungen ist.

Des Weiteren ist hierin auch ein Verfahren zur Herstellung eines Antikörpers beschrieben, umfassend:

(a) Durchführung der Schritte der weiter oben beschriebenen Herstellungs- und Identifizierungsverfahren;

(b) Einbringen des MUC1-Moleküls, der Zelle oder der Zelllysate in ein Tier; und

(c) Bereitstellen eines Antikörpers, der das MUC1-Molekül spezifisch erkennt und der einen besonders starken additiven Effekt zeigt

In diesem Kontext ist der Ausdruck "besonders starker additiver Effekt" als solcher zu verstehen, dass die Erhöhung der Bindung des Antikörpers gegenüber multiplen PDTR-glykosylierten Tandem Repeats wie unter 4) beschrieben, im Vergleich zu kurzen PDTR-glykosylierten MUC1 Peptiden, wie unter 2) beschrieben, und beide im Vergleich zu nicht PDTR-glykosylierten kurzen MUC1-Peptiden stärker ist als die Erhöhungen der Bindung der Antikörper A76-A/C7, VU-11E2, VU-11D1, BC4E549, VU-12E1, VU-3D1 und b-12 (oder vergleichbar zu den stärksten Erhöhungen dieser Antikörper ist).

Die Definitionen der Begriffe, die zuvor gemacht wurden, treffen mutatis mutandis auf diese und die nachfolgenden Ausführungsformen zu.

Unter dem Begriff "Antikörpern" versteht man:

- murine, chimäre, humanisierte, humane Antikörper
- ganze Antikörper verschiedener Isotypen oder Antikörperfragmente, beispielsweise single-chain Antikörperfragmente (ScFv), Fäb-Fragmente, multivalente Antikörperfragmente (beispielsweise Dia-, Tria-, Tetrabodies), oder Antikörperfragmente anderer Formate, auch solche, die als Fusionsproteine mit andere Peptiden oder Proteinstrukturen gekoppelt verwendet werden.
- Antikörper oder Antikörperfragmente, die neben der erfindungsgemäßen Spezifität für MUC1 noch eine oder mehrere weitere Spezifitäten besitzen, beispielsweise bispezifische Antikörper.
- Moleküle, die streng genommen keine Antikörper sind, jedoch identische Bindungseigenschaften, wie die erfindungsgemäßen Antikörper, die das MUC1-Tumorepitop tragen, aufweisen, und / oder Teile von Antikörpern, beispielsweise CDR-Regionen oder Teile davon tragen, wie beispielsweise Affibodies oder andere Trägerstrukturen, die Bindungsdomänen zur spezifischen Bindung tragen. Die Erfindung betrifft auch Antikörper, die von den hier beschriebenen Antikörpern abgeleitet werden und dadurch einen additiven Bindungseffekt bekommen, erhöhen und/oder besser gegenüber multiplen PDTR-glykosylierten Tandem Repeats binden als der Ausgangsantikörper. Eine entsprechende Ableitung des Antikörpers kann durch an sich bekannte Methoden durch gezielte oder zufällige Veränderungen des Antikörpers erfolgen und wird mit Hilfe der vergleichenden Bindung an entsprechende MUC1-Glykopeptide wie unter den Beispielen 1 bis 3 getestet.

Die Bereitstellung dieser Antikörper erfolgt nach an sich bekannten Techniken. Die Antikörper können als polyklonale Antikörper direkt aus dem Serum der Tiere gewonnen werden oder durch die bekannten Verfahren zur Herstellung monoklonaler Antikörper als solche bereitgestellt werden (Beispiel 6).
Die durch das hierin beschriebene Verfahren hergestellten Antikörper können vorteilhafterweise gegen das MUC1-Tumorepitop zur Behandlung von Tumorerkrankungen verwendet werden. Diese Antikörper können in der Behandlung oder Prophylaxe von Tumorerkrankungen direkt eingesetzt werden oder mit Effektorstrukturen gekoppelt werden. Unter Effektorstrukturen versteht man erfindungsgemäß solche chemischen oder biochemischen Verbindungen, Moleküle oder Atome, die direkt oder indirekt eine Abtötung oder Schädigung, einschließlich beispielsweise Wachstumsverlangsamung oder Wachstumsinhibition von Tumorzellen bewirken. Hierzu gehören beispielsweise: Radioisotope, Toxine, Cytostatika und andere Effektormoleküle wie beispielsweise Cytokine und Chemokine oder andere Strukturen, die selbst Effektoren darstellen oder an die Effektormoleküle gekoppelt werden, beispielsweise mit Toxinen oder Cytostatika beladene Liposomen, die MUC1-Antikörper tragen. Beim letzteren Beispiel sind gerade auch solche Effektorstrukturen gemeint, die neben dem MUC1-Antikörper für die Tumorspezifität auch solche Moleküle tragen, die für eine Aufnahme der Effektorstrukturen oder Teile davon in die Zellen verantwortlich sind, wie beispielsweise Antikörper gegen Rezeptoren, die eine Rezeptor-vermittelte Endozytose bewirken und damit die MUC1-vermittelte Aufnahme verbessern. Die Verbindung der Antikörper mit den Effektorstrukturen erfolgt nach an sich bekannten Methoden. Die Kopplungen können dabei beispielsweise direkt durch kovalente oder nicht-kovalente Beladung erfolgen, durch chemische Kopplung, wobei ein zusätzliches chemisches oder biologisches Molekül notwendig sein kann, beispielsweise ein Chelator oder ein Linker, oder in Form von Fusionsproteinen oder - peptiden durch Fusion.
Eingesetzt werden die Antikörper bei der Behandlung von Tumorerkrankungen mit MUC1-tragenden Tumorzellen oder zur Prophylaxe, die beispielsweise die Ausbildung von primären Tumoren oder Metastasen verhindert. Bevorzugtes Ziel ist dabei die Behandlung der minimalen residualen Erkrankung und von Metastasen. Die Antikörper werden dabei in einer geeigneten Formulierung einmalig oder wiederholt in geeigneten zeitlichen Abständen und Dosen verabreicht. Eine bevorzugte Form sind radioaktiv markierte Antikörper. Eine weitere bevorzugte Form sind radioaktiv markierte Dia-Tria-, Tetrabodies. Weitere bevorzugte Formen sind radioaktiv markierte single chain-Antikörperfragmente und chimäre Antikörper.
In Verfahren zur Tumordiagnostik und Prognose werden MUC1-spezifische Antikörper, die das hierin beschriebene Tumorepitop erkennen, in an sich bekannten Verfahren eingesetzt, um MUC1 im Serum oder in Gewebspräparaten nachzuweisen. Dabei werden freies MUC11 in Immunkomplexen vorliegendes MUC1 und auf Zellen gebundenes MUC1 nachgewiesen und das Vorhandensein der tumorassoziierten MUC1-Moleküle qualitativ, quantitativ und/ oder in relativen Quantitäten nach an sich bekannten Methoden bestimmt. Die gleichen Verfahren werden auch zur Verlaufskontrolle von Tumorerkrankungen und zur Kontrolle von Behandlungsverläufen eingesetzt. Die in den Verfahren verwendeten Methoden sind an sich bekannt, beispielsweise ELISA, Western-Blot, FACS (Fluoreszenaktiviertes Cellsorting), MACS (magnet vermittelte Zellsortierung), ADCC (Antikörpervermittelte Zellzytotoxizität), CDC (Komplement vermittelte Zytotoxicität), Immuncytochemie und Immunhistochemie. Ein Beispiel hierfür ist der weiter unten beschrieben erfindungsgemäße Sandwich-ELISA mit den dort beschriebenen Antikörpern und in den Beispielen beschriebene Methoden. In einem bevorzugten Verfahren werden dabei vergleichend Antikörper verwendet, die das hierin beschriebene Tumorepitop erkennen, mit solchen Antikörpern, die das Tumorepitop nicht erkennen, um eine bessere Differenzierung und Einschätzung zu erreichen. Dies dient der Diagnose von MUC1-positiven Tumoren, des Monitorings des Verlaufs der Tumorerkrankung und der Prognose. Die Bindung der Antikörper bestimmt ebenfalls den Anteil an immunsuppressivem MUC1, da MUC1, das von Antikörpern gegen das erfindungsgemäße Tumorepitop gebunden wird, keine oder eine verringerte Immunsuppressivität aufweist und das Immunsystem in geeigneten Formulierungen spezifisch gegen Tumor-MUC1 aktivieren kann.
In einem entsprechenden Verfahren werden die Antiköper, die das hierin beschriebene Tumorepitop und solche, die durch das erfindungsgemäße Verfahren hergestellt wurden, erkennen, zu einer in vivo Diagnostik verwendet. Hierfür werden die Antikörper mit geeigneten an sich bekannten Verfahren markiert und somit für an sich bekannte bildgebende Verfahren am Menschen zugänglich gemacht, beispielsweise Radioimmunodiagnostik, PET-Scan-Verfahren oder Immunofluoreszenzendoskopie, beispielweise durch Kopplung und/ oder Beladung mit entsprechenden Molekülen, beispielsweise radioaktiven Isotope, beispielsweise das Indium, oder Fluoreszenzfarbstoffe beispielsweise dem Cy3, Cy2, Cy5 oder FITC.

Ferner betrifft die Erfindung ein Verfahren zur Herstellung eines Arzneimittels umfassend die Schritte der erfindungsgemäßen Verfahren und weiterhin umfassend den Schritt der Formulierung des MUC1-Moleküls in pharmazeutisch verträglicher Form.

Unter dem Begriff "Arzneimittel" sind erfindungsgemäß Stoffe und Zubereitungen aus Stoffen definiert, die dazu bestimmt sind, durch Anwendung am oder im menschlichen Körper Krankheiten, Leiden, Körperschäden oder krankhafte Beschwerden zu heilen, zu lindern oder zu verhüten. Während des erfindungsgemäßen Herstellungsverfahrens können den mit den erfindungsgemäßen Verfahren identifizierten Verbindungen medizinische und/oder pharmazeutisch-technische Hilfsstoffe zugesetzt werden. Medizinische Hilfsstoffe sind erfindungsgemäß solche Stoffe, die zur Produktion (als aktive Ingredienzien) von Arzneimitteln in einem erfindungsgemäßen Verfahren eingesetzt werden. Pharmazeutisch-technische Hilfsstoffe dienen lediglich der geeigneten Formulierung des Arzneimittels und können sogar, sofern sie nur während des Verfahrens benötigt werden, anschließend entfernt werden oder können als pharmazeutisch verträgliche Träger Teil des Arzneimittels sein. Beispiele für pharmazeutisch verträgliche Träger sind nachstehend aufgeführt.
Die Arzneimittelformulierung erfolgt gegebenenfalls in Kombination mit einem pharmazeutisch verträglichen Träger und/oder Verdünnungsmittel.
Beispiele für geeignete pharmazeutisch verträgliche Träger sind dem Fachmann bekannt und umfassen Phosphatgepufferte Kochsalzlösungen, Wasser, Emulsionen wie z.B. Öl/Wasser-Emulsionen, verschiedene Arten von Detergenzien, sterile Lösungen, etc. Arzneimittel, die solche Träger umfassen, können mittels bekannter konventioneller Methoden formuliert werden. Diese Arzneimittel können einem Individuum in einer geeigneten Dosis verabreicht werden, z.B. in einem Bereich von 1μg bis 100 mg pro Tag und Patient. Die Verabreichung kann auf verschiedenen Wegen erfolgen, z.B. direkt auf der Haut, intravenös, intraperitoneal, subkutan, intramuskulär, lokal oder intradermal. Die Verabreichung von Nukleinsäuren kann auch in Form von Gen-Therapie geschehen. Die Art der Dosierung wird vom behandelnden Arzt entsprechend den klinischen Faktoren bestimmt. Es ist dem Fachmann bekannt, daß die Art der Dosierung von verschiedenen Faktoren abhängig ist, wie z.B. der Größe, der Körperoberfläche, dem Alter, dem Geschlecht oder der allgemeinen Gesundheit des Patienten, aber auch von dem speziellen Mittel, welches verabreicht wird, der Dauer und Art der Verabreichung und von anderen Medikamenten, die möglicherweise parallel verabreicht werden. Erfindungsgemäße Tumormittel umfassen eine pharmakologische Substanz, die ein oder mehrere erfindungsgemäße tumorassoziierte MUC1-Moleküle, Zellen, die diese umfassen oder aus diesen Zellen hergestellte Zelllysate in einer geeigneten Lösung oder Verabreichungsform enthält. Diese können entweder alleine oder in Kombination mit einem oder mehreren Adjuvantien oder einem anderen geeigneten Stoff zur Wirkungsverstärkung verabreicht werden. Als bevorzugte Adjuvantien werden QS-21, GPI-0100 oder andere Saponine, Wasser-Öl Emulsionen wie beispielsweise Montanide Adjuvantien, Polylysin, Polyargininverbidnungen, DNA-Verbindungen wie beispielsweise CpG, Detox, Bakterielle Vakzinen wie beispielsweise Typhusvakzine oder BCG-Vakzinen verwendet und in einer entsprechend geeigneten Weise nach an sich bekannten Methoden mit den erfindungsgemäßen tumorassoziierten MUC1-Molekülen und/ oder Antikörper gemischt. Die Herstellung der Tumormittel erfolgt nach an sich bekannten Methoden. Erfindungsgemäß ist ein Tumormittel auch eine Kombination von 2 oder mehreren der erfindungsgemäßen Tumormittel, sowie eine Kombination mit anderen Tumorvakzinen oder Tumorbehandlungen, wie beispielsweise Antikörpertherapien, Chemotherapien oder Radiotherapien die auf eine geeignete Weise zeitlich gemeinsam oder getrennt verabreicht beziehungsweise angewandt werden. Herstellung der Tumormittel erfolgt nach an sich bekannten Methoden.
Die erfindungsgemäße Verwendung der Tumormittel ist zur Behandlung von MUC1-positiven Tumorerkrankungen, wie beispielsweise Mammakarzinome, Ovarialkarzinome, Kolonkarzinome, Gastrointestinalkarzinome, Pankreaskarzinome, Lungenkarzinom, multiples Myelom. Die Behandlung geht beispielsweise gegen Primärtumoren, minimal residuale Tumorerkrankungen, Metastasen auch als adjuvante Behandlung. Die erfindungsgemäße Verwendung der Tumormitel ist ebenfalls zur Prophylaxe von MUC1-positiven Tumorerkrankungen. Die prophlaktische Anwendung zielt beispielsweise auf eine Prophylaxe des Tumors sowie von Metastasen. Die Tumormittel werden in einer geeigneten Form nach an sich bekannten Methoden verabreicht. Eine bevorzugte Variante ist die Injektion beziehungsweise Verabreichung der Tumorvakzine subkutan, intradermal, systemisch intravenös, intraperitoneal, intrarectal, lokal in Körperkavitäten, wie beispielsweise dem Peritoneum, oder lokal direkt in Organe oder Lymphknoten. Verabreichungsarten können bevorzugterweise auch kombiniert werden, wobei sie an verschiedenen Behandlungstagen der an einem Behandlungstag verabreicht werden können. Dabei können erfindungsgemäß auch 2 oder mehrere der erfindungsgemäßen Tumormittel kombiniert werden oder eine oder mehrere erfindungsgemäße Tumormittel mit ein oder mehreren Tumormittel oder Tumorbehandlungen, wie beispielsweise Antikörpertherapien, Chemotherapien oder Radiotherapien die zeitlich gemeinsam oder getrennt verabreicht beziehungsweise angewandt werden.

Das erfindungsgemäße Verfahren umfasst auch die Herstellung eines Tumormittels, das eine Zellvakzine enthält, die durch die Beladung von dendritischen Zellen nach an sich bekannten Methoden mit den erfindungsgemäßen tumorassoziierten MUC1-Molekülen und gegebenenfalls anschließender Reifung der Zellen erhältlich ist. Die so gewonnene Zellvakzine kann nach an sich bekannten Methoden verabreicht werden. Beispielsweise werden unreife dendritische Zellen mit tumorassoziierten MUC1-Molekülen nach an sich bekannten Methoden versetzt. Die dendritischen Zellen nehmen die tumorassoziierten MUC1-Moleküle auf, prozessieren sie und präsentieren Fragmente davon auf ihrer Oberflache im Kontext mit MHC-Molekülen und costimulatorischen Molekülen. Nach einer weiteren Reifung nach an sich bekannten Methoden werden die Zellen in einer geeigneten Formulierung am Menschen angewandt. Ein weiteres Beispiel ist die Beladung der reifen dendritischen Zellen nach an sich bekannten Methoden des "Pulsing". Bei den dendritischen Zellen handelt es sich um autologe, allogene oder semiallogene dendritische Zellen beziehungsweise deren Vorläuferzellen oder Zellen aus Zelllinien, die die funktionellen Eigenschaften von dendritischen Zellen besitzen, die ex vivo nach an sich bekannten Methoden entsprechend geeignete Behandlung zur Entwicklung und Reifung bekommen. Die Herstellung dieser Tumorvakzinen mit geeigneten dendritischen Zellen und erfindungsgemäßen MUC1-Molekülen erfolgt nach an sich bekannten Methoden (siehe Beispiel 7). Erfindungsgemäß besteht eine weitere Variante der Tumormittel in einer T-Zell-Therapie, bei der T-Zellen, die geeignete erfindungsgemäße tumorassoziierte MUC1-Moleküle, einschließlich Fragmente derer, oder prozessierter Derivate, die solchen die durch die Prozessierung und Präsentation in vivo entsprechen, im Kontext mit MHC-Klasse-Molekülen und costimulatorischen Molekülen erkennen und bei der Präsentation über eine geeignete Antigenpräsentierende Zellen aktiviert werden. Die Herstellung solcher T-Zell-Therapien erfolgt nach an sich bekannten Methoden. (siehe Beispiel 7)

In einer besonders bevorzugten Ausführungsform des Verfahrens werden Zelllysate von Zellen, die für tumorassoziierte MUC1-Moleküle auf den Zellen angereichert wurden, hergestellt und in den hier beschriebenen Anwendungen in der Tumorbehandlung und -prophylaxe eingesetzt. Die erfindungsgemäßen Verfahren zur Anwendung am Menschen beinhaltet geeignete pharmakologische Formulierungen, bevorzugt in Kombination mit Adjuvantien und/ oder costimulatorischen Molekülen, wie beispielsweise GM-CSF. Eine hierbei bevorzugte Variante ist die Beladung von dendritischen Zellen oder Zellen, die eine immunstimulatorische Funktion wie dendritische Zellen besitzen, mit Zelllysaten und die Verabreichung von diesen Zellen, bevorzugt nach Bestrahlung, am Menschen nach an sich bekannten Methoden. Die Behandlung mit beladenen dendritischen Zellen und Zelllysaten wird vorzugsweise kombiniert, wobei spätere Boosterungen bevorzugt mit Zelllysaten in geeigneten erfindungsgemäßen pharmakologischen Formulierungen, bevorzugt mit Adjuvantien oder costimulatorischen Molekülen (beispielsweise GM-CSF) durchgeführt werden.

Die bevorzugte Verwendung der Zelllysate oder mit Zelllysaten beladene dendritische Zellen ist zur Behandlung und Prophylaxe von MUC1-positiven Tumorerkrankungen, wie beispielsweise Mammakarzinome, Ovarialkarzinome, Colonkarzinome, Gastrointestinalkarzinome, Pankreaskarzinome, Lungenkarzinom, multiples Myelom, und dabei gegen Primärtumoren, minimal residuale Tumorerkrankungen, Metastasen, und als adjuvante Behandlung. Bevorzugterweise werden die Zelllysate oder mit Zelllysaten beladene dendritische Zellen nach an sich bekannten Methoden subkutan, intradermal, intrarectal, intranodal (Lymphknoten) oder systemisch verabreicht.

Die zuvor eingeführten Definitionen der Begriffe sind für die Begriffe in den nachfolgend beschriebenen Ausführungsformen mutatis mutandis anwendbar.

Die vorliegende Erfindung betrifft weiterhin ein Verfahren zur Herstellung eines Diagnostikums umfassend die Schritte der erfindungsgemäßen Verfahren und weiterhin umfassend den Schritt der Formulierung des MUC-1-Moleküls in eine diagnostisch verwendbare Form.

Unter dem Begriff "Diagnostikum" sind erfindungsgemäß Stoffe und Zubereitungen aus Stoffen definiert, die dazu bestimmt sind, durch Anwendung am oder im menschlichen Körper oder Teilen davon Krankheiten, Leiden, Körperschäden oder krankhafte Beschwerden zu erkennen. Als Teile des menschlichen Körpers sind vorzugsweise Körpergewebsproben oder Körperflüssigkeiten, wie Blut, Lymphe, Urin, Spinalflüssigkeit oder Sperma, oder Gewebebiopsien zu verstehen. Die Formulierung des Diagnostikums umfasst vorzugsweise die Modifikation der hergestellten MUC1-Moleküle mit Substanzen, die einen Nachweis des Moleküls erlauben. Geeignete Substanzen sind im Stand der Technik bekannt. Ausgehend von der Wahl der Substanz ist der Fachmann in der Lage geeignete Maßnahmen zur Formulierung des Diagnostikums einzusetzten.

Für die Diagnostik können erfindungsgemäß auch Substanzen nach an sich bekannten Methoden an die tumorassoziierten MUC1-Moleküle gekoppelt werden, die einen Nachweis der MUC1-spezifischen Antikörper erleichtern, beispielsweise durch Biotinylierung der tumorassoziierten MUC1-Moleküle und anschließenden Immobilisierung auf ELISA Platten.

In einem weiteren erfindungsgemäßen Verfahren zur Tumordiagnostik und Prognose werden erfindungsgemäße tumorassoziierte MUC1-Moleküle, die das Tumorepitop tragen, zur Bestimmung von MUC1-Antikörpern im Serum von Menschen verwendet, die das Tumorepitop erkennen. In einem bevorzugten Verfahren werden dabei vergleichend solche MUC1-Moleküle oder Fragmente davon verwendet, die das erfindungsgemäße Tumorepitop tragen, mit solchen Antikörpern, die das Tumorepitop nicht tragen, um eine bessere Differenzierung und Einschätzung zu erreichen. Dabei werden erfindungsgemäß freie Antikörper und in Immunkomplexen vorliegende Antikörper nachgewiesen und ihre Spezifitäten gegenüber dem erfindungsgemäßen MUC1-Tumorepitop und bevorzugt anderen Epitopen auf dem MUC1, einschließlich Kohlenhydratantigenen qualitativ, quantitativ und/ oder in relativen Quantitäten nach an sich bekannten Methoden bestimmt. Die gleichen Verfahren werden erfindungsgemäß auch zur Verlaufskontrolle von Tumorerkrankungen und zur Kontrolle von Behandlungsverläufen einschließlich des Monitorings von Immunantworten, beispielsweise IgG- und IgM-Antikörperantworten gegen unterschiedliche Epitope einschließlich des MUC1-Tumorepitopes auf MUC1, zur Kontrolle und Dosierung von Tumorbehandlungen eingesetzt. Die in den Verfahren verwendeten Methoden sind an sich bekannt, beispielsweise ELISA, Westerblot, FACS (Fluoreszenaktiviertes Cellsorting), MACS (magnet vermittelte Zellsortierung), ADCC (antikörpervermittelte Zellzytotoxizität), CDC (Komplementvermittelte Zytotoxizität), Immuncytochemie und Immunhistochemie. Ein Beispiel hierfür sind Glykopeptide gekoppelt an ein Trägermolekül oder Biotin immobilisiert in einem ELISA, in dem die Bindung von Serum-Antikörpern aus Menschen durch anti-human- IgG oder anti-human-IgM spezifisch nach an sich bekannter Detektionsmethode nachgewiesen, quantifiziert und damit in ihrer Spezifität bestimmt werden. Ein solches Verfahren wird beispielsweise angewendet um den natürlichen Antikörpertiter von MUC1-spezifischen Antikörpern im Serum zu bestimmen und dabei in die unterschiedlichen Spezifitäten zu untergliedern. Dies ist beispielsweise geeignet für die Tumorprognose von Brustkrebs. Patientinnen mit einem hohen Titer an Antikörpern gegen das erfindungsgemäße MUC1-Tumorepitop haben eine deutlich verbesserte Prognose gegenüber solchen mit einem niedrigeren Titer gegen das erfindungsgemäße MUC1-Tumorepitop.

Die zuvor eingeführten Definitionen der Begriffe sind für die Begriffe in den nachfolgend beschriebenen Ausführungsformen mutatis mutandis anwendbar.

Weiterhin betrifft die vorliegende Erfindung die Verwendung eines MUC1-Moleküls, einer Zelle, eines Zelllysates oder eines Antikörpers erhältlich durch ein erfindungsgemäßes Verfahren zur Herstellung eines Arzneimittels zur Prävention oder Behandlung von Tumoren.

Vorzugsweise ist das Arzneimittel im Rahmen der erfindungsgemäßen Verwendung ein Impfstoff.

Aus den oben gemachten Ausführungen ergibt sich, dass die vorliegende Erfindung weiterhin die Verwendung eines Antikörpers erhältlich durch ein erfindungsgemäßes Verfahren zur Herstellung eines Diagnostikums zur Diagnose von Tumoren betrifft.

Schließlich umfasst die vorliegende Erfindung ein gereinigtes tumorassoziiertes MUC1-Molekül, welches im Menschen immunstimulatorisch wirkt und durch ein erfindungsgemäßes Verfahren erhältlich ist.

Der Begriff "gereinigt" definiert ein MUC1-Molekül, wie es durch das erfindungsgemäße Verfahren hergestellt werden kann. Ein gereinigtes tumorassoziiertes MUC1-Molekül umfasst daher auch eine Gruppe von bestimmten MUC1-Glykoproteinen, die sich dadurch auszeichnen, dass sie im Menschen in der Lage sind, eine Immunantwort auszulösen, jedoch wenig oder, bevorzugterweise gar nicht immunsuppressiv wirken. Weitere funktionelle und strukturelle Eigenschaften des erfindungsgemäßen gereinigten MUC1-Moleküls sind ebenso in den nachfolgenden Beispielen beschrieben.

Die Figuren zeigen:

**Figur 1** zeigt Beispiele für die Bindungsmuster verschiedener anti-MUC1-Antikörper. Insgesamt wurden 4 Verhaltensmuster definiert:

1) GD-1: Antikörper, die am PDTR-Motiv erst nach dessen Glykosylierung binden (GD steht für glycosylation-dependent). Beispiel: A76-A/C7 (Abb.1A).

2) GD-2: Antikörper, deren Bindung durch Glykosylierung am PDTR-Motiv verbessert wird. Beispiel: VU-3D1 (Abb.1B).

3) iGD: Antikörper, deren Bindung durch Glykosylierung am PDTR-Motiv verringert oder unterbunden wird (inverse glycosylation-dependency). Beispiel: VU-4H5 (Abb.1B).

4) GI: Antikörper, deren Bindung an die PDTR-Region von deren Glykosylierung unabhängig ist (glycosylation-independent). Beispiel: HMFG-1 (Abb.1B)

**Figur 2** zeigt Beispiele für Bindungsmuster von anti-MUC1-Antikörpern gegenüber nicht glykosylierten MUC1-Peptiden verschiedener Länge (1-6 Tandem-Repeats).

Die Ergebnisse zeigen, dass Antikörper, die durch eine Immunisierung von Mäusen mit Tumormaterial erzeugt wurden,

- Positiv abhängig von der Glykosylierung in der PDTR-Region kurzer MUC1-Peptide(GD-1 und GD-2) und

- Positiv abhängig von der zunehmenden Länge nichtglykosylierter MUC1-Peptide und damit der Zahl der Tandem Repeats sind

Antikörper, die durch eine Immunisierung von Mäusen mit Nicht-Tumormaterial erzeugt wurden,

- unabhängig (GI) oder negativ abhängig (invers abhängig; iGD) von der Glykosylierung in der PDTR-Region kurzer MUC1-Peptide sind (GI) oder

- unabhängig von der Länge der MUC1-Peptide und damit der Zahl der Tandem Repeats sind

**Figur 3** zeigt 2 Beispiele für die Bindung von MUC1-spezifischen monoklonalen Antikörpern an glykosylierte MUC1-Peptide unterschiedlicher Länge (= unterschiedlicher Anzahl an Tandem-Repeats).

**Figur 4** zeigt die Bindung des Antikörpers VU-4H5 an glykoslyierte MUC1-Peptide unterschiedlicher Länge.

**Figur 5A**: Immuncytochemische Färbung von ZR-75-1 Zellen (1) und Zellen der Subzellinie (2) mit dem Antikörper A76-A/C7.

**Figur 5B:** Sekretion von A76-A/C7 positivem MUC1 von ZR-75-1 sub und MCF-7 Zellen. Das sekretierte MUC1 wurde 3, 4, 5, 6 und 7 Tage nach Aussäen von $10^5$ Zellen im Zellkulturüberstand durch ELISA mit den HMFG1 und A76-A/C7 Antikörpern nachgewiesen. Zu beachten sind die unterschiedlichen Verdünnungen der Zellkulturüberstände.

**Figur 5C**: Expressionsvektor zur Integation der MUC1-cDNA unter Kontrolle des CMV Promoters in das ZR-75-1 Genom sowie die MUC1-cDNA Sequenz.

**Figur 5D**: Rec site Vektor zur Integration der Rekombinationsstelle in das ZR-75-1 Genom und Darstellung der Methode der Integration.

**Figur 5E:** SDS-Polyacrylamidgelanalyse verschiedener MUC1-Präparationen.

**Figur 5F:** Das gereinigten sezernierten tumorassoziierten MUC1 zeigen keinerlei immunsupressiven Effekte auf T-Zellen *in vitro.*

**Figur 6:** Stimulierung von naiven T Zellen mit Hilfe von mit dem refindungsgemäßen MUC1-beladenen effektiven dendritischen Zellen und deren MUC1-spezifische anti-Tumor Aktivität (Details siehe Beispieltext).

Die Erfindung wird nun illustriert durch die nachfolgenden Beispiele. Die Beispiele dienen ausschließlich der Erläuterung und beschränken den Schutzumfang der Erfindung nicht.

**Beispiel 1: Bindungsmuster verschiedener anti-MUC1-Antikörper an ein MUC1-Peptid in Abhängigkeit von der Glykosylierung des PDTR-Motivs**

Monoklonale anti-MUC1-Antikörper (Maus) wurden im Enzym-Immunoassay (ELISA) auf ihre Bindungsfähigkeit gegen synthetische MUC1-Peptide und -Glykopeptide mit folgender Sequenz getestet: Biotin-APPAHGVTSAPDT(GalNAc)RPAPGSTAPPAHGVTSA. Als Kontrolle diente das gleiche, aber nicht-glykosylierte Peptid. Die Antigene wurden auf Streptavidinbeschichteten Mikrotestplatten (BioTeZ, Berlin) in einer Konzentration von 0,5 μg/ml (100 pl/Well) immobilisiert. Nach dreimaligem Waschen mit PBS/0,05 % Tween 20 wurden diverse gereinigte anti-MUC1-Antikörper in Verdünnungsreihen aufgetragen und für 2 h bei 37 °C inkubiert. Als Zweitantikörper diente Kaninchen-anti-Maus-Ig, peroxidase-markiert (Dako, Hamburg) in einer Verdünnung von 1:4000 (1,5 h, 37 °C).Nach dreimaligem Waschen wurde die Platte mit Ortho-phenylendiamin entwickelt und die Farbreaktion mit 2,5 N Schwefelsäure gestoppt. Die OD wurde bei 492 nm gemessen.

Figur 1 zeigt Beispiele für die Bindungsmuster verschiedener anti-MUC1-Antikörper. Insgesamt wurden 4 Verhaltensmuster definiert:

1) GD-1: Antikörper, die am PDTR-Motiv erst nach dessen Glykosylierung binden (GD steht für glycosylation-dependent). Beispiel: A76-A/C7 (Abb.1A).

2) GD-2: Antikörper, deren Bindung durch Glykosylierung am PDTR-Motiv verbessert wird. Beispiel: VU-3D1 (Abb.1B).

3) iGD: Antikörper, deren Bindung durch Glykosylierung am PDTR-Motiv verringert oder unterbunden wird (inverse glycosylation-dependency). Beispiel: VU-4H5 (Abb.1B).

4) GI: Antikörper, deren Bindung an die PDTR-Region von deren Glykosylierung unabhängig ist (glycosylation-independent). Beispiel: HMFG-1(Abb.1B)

Tabelle 1 enthält eine Übersicht der Bindungsmuster eines Panels von monoklonalen anti-PDTR(MUC1)-Antikörpern mit PDTR-glykosylierten und unglykosylierten MUC1-Peptiden. Die Tabelle ist in zwei Untertabellen (A) und (B) unterteilt. Tabelle 1A beinhaltet Antikörper, die durch eine Immunisierung mit MUC1 aus Tumormaterial durchgeführt wurden. Tabelle 1 B beinhaltet Antikörper, die durch eine Immunisierung mit MUC1 aus Nicht-Tumormaterial durchgeführt wurden.

Tabelle 1:

Antikörper gegen die immundominante PDTR-Region des MUC1:

Bindungsmuster gegenüber am PDTR glykosylierten und nicht-glykosylierten MUC1-Peptiden in Abhängigkeit von der Art des Immunogens

A. MUC1 aus Tumormaterial

| Antikörper | Immunogen | Bindungsmuster | | | |
|---|---|---|---|---|---|
| | | GD-1 | GD-2 | iGD | GI |
| A76-A/C7 | Tumorzellinie T-47D | + | | | |
| VU-11E2 | Tumorzellinie ZR75-1 | + | | | |
| VU-11D1 | Tumorzellinie ZR75-1 | + | | | |
| Ma552 | Tumorzellinie ZR75-1 | | + | | |
| VU-3C6 | Tumorzellinie ZR75-1 | | + | | |
| BC4E549 | Membranpräparation der Tumorzellinie T-47D | | + | | |
| VU-12E1 | Tumorzellinie ZR75-1 | | + | | |
| VU-3D1 | Tumorzellinie ZR75-1 | | + | | |
| b-12 | Mischung mehrer Tumorzellinien | | + | | |
| B27.29 | MUC1 aus Tumor Ascites | | + | | |
| MF 06 | MUC1 aus Ovarialcyste | | + | | |

B. MUC1-Peptide (unglykolysiert) oder Präparationen aus Nicht-Tumormaterial

| Antikörper | Immunogen | Bindungsmuster | | | |
|---|---|---|---|---|---|
| | | GD-1 | GD-2 | iGD | GI |
| VU-4H5 | 60-mer, BSA konjugiert | | | + | |
| HMPV | HMFG aus Frauenmilch | | | + | |
| HMFG-1 | HMFG aus Frauenmilch | | | | + |
| BC2 | HMFG aus Frauenmilch | | | | + |
| VA2 | 100-mer Fusionsprotein | | | | + |
| Mc5 | HMFG aus Frauenmilch | | | | + |
| E29 | HMFG aus Frauenmilch | | | | + |
| BC3 | HMFG aus Frauenmilch | | | | + |
| 214D4 | MUC1-Peptid (Fusionsprotein) | | | | + |
| BCP8 | MUC1-Peptid | | | | + |
| VA1 | 100-mer Fusionsprotein | | | | + |
| C595 | MUC1 aus Urin | | | | + |
| BC4W 154 | HMFG aus Frauenmilch | | + | | |

**Beispiel 2: Bindung von MUC1-spezifischen Antikörpern gegen die immundominante PDTR-Region an nicht-glykosylierte MUC1-Peptide unterschiedlicher Länge (nicht-glykosylierte multiple Tandem-Repeats)**

Die MUC1-Peptide der folgenden Sequenz in unterschiedlicher Länge (Öligomerisierung)

[VTSAPDTRPAPGSTAPPAHG]$_n$ ; n = 1-6
wurden in einer Konzentration von mit 0,5μg/Well in Coating-Puffer bei 37 °C über Nacht auf einer 96-Well-TC-Mikrotiterplatte durch Antrocknen immobilisiert. Die Platten wurden gewaschen (PBS/ 0,05% Tween20) und anschließend für 2 h mi t 50 μl/Well gereinigtem Antikörper in einer Konzentration von 10μg/ml inkubiert. Nach dreimaligem Waschen wurde die Platte mit Peroxidase-markiertem polyklonalem Kaninchen-anti-Maus Immunglobulin-Serum (P260, Dako) in einer Verdünnung von 1:2000 inkubiert. Nach dreimaligem Waschen wurde die Platte mit o-Phenylendiamin entwickelt und die Farbreaktion mit 2,5 N Schwefelsäure gestoppt. Die OD wurde bei 492 nm bestimmt.

Die Anzahl der Tandem-Repeats pro Ansatz wurde in den Experimenten dadurch konstant gehalten, dass von allen verglichenen Peptiden gleiche Gewichtsmengen pro Well eingesetzt wurden.

Die Ergebnisse sind in Tabelle 2 zusammengefasst:

Die Klassifizierung wurde nach dem Verhältnis der OD 100-mer/20-mer = X wie folgt vorgenommen:

Ohne nennenswerte Bindung an das 20-mer, jedoch einer deutlichen Bindung an 100-mer: Gruppe LD1 (absolut längenabhängig)

X > 3,0 : Gruppe LD2 (stark längenabhängig)

X = 1,5 - 3,0 : Gruppe LD3 (mässig längenabhängig)

X < 1,5 : Gruppe LI (längenunabhängig)

Weiterhin sind in die Tabelle die Angaben über die Glykosylierungsabhängigkeit aus Tab.1 zum Vergleich mit aufgenommen worden.

Tabelle 2:

Bindungsmuster monoklonaler anti-MUC1-Antikörper gegen das immundominante PDTR-Motiv: Bindungsmuster gegenüber am PDTR glykosylierten und nichtglykosylierten MUC1-Peptiden in Abhängigkeit von der Art des Immunogens und mit nicht glykosylierten MUC1-Peptiden unterschiedlicher Länge (1-6 Tandem-Repeats).

Antikörper Bindungsmuster in Abhängigkeit von:

A. MUC1 aus Tumormaterial

| | Glykosylierung | | | | Länge | | | |
|---|---|---|---|---|---|---|---|---|
| | GD-1 | GD-2 | iGD | GI | LD-1 | LD-2 | LD-3 | LI |
| A76-A/C7 | + | | | | + | | | |
| VU-11 E2 | + | | | | + | | | |
| VU-11D1 | + | | | | + | | | |
| Ma552 | | + | | | + | | | |
| VU-3C6 | | + | | | + | | | |
| BC4E549 | | + | | | | + | | |
| VU-12E1 | | + | | | | + | | |
| VU-3D1 | | + | | | | | + | |
| b-12 | | + | | | | | + | |
| B27.29 | | + | | | | | + | |
| MF 06 | | + | | | | | + | |

B. MUC1-Peptide (unglykosyliert) oder Präparationen aus Nicht-Tumormaterial

| | Glykosylierung | | | | Länge | | | |
|---|---|---|---|---|---|---|---|---|
| | GD-1 | GD-2 | iGD | GI | LD-1 | LD-2 | LD-3 | LI |
| VU-4H5 | | | + | | | + | | |
| HMPV | | | + | | | + | | |
| HMFG-1 | | | | + | | + | | |
| BC2 | | | | + | | + | | |
| VA2 | | | | + | | + | | |
| Mc5 | | | | + | | + | | |
| E29 | | | | + | | | + | |
| BC3 | | | | + | | | + | |
| 214D4 | | | | + | | | + | |
| BCP8 | | | | + | | | + | |
| VA1 | | | | + | | | + | |
| C595 | | | | + | | | | + |
| BC4W154 | | + | | | | | | + |

Figur 2 zeigt Beispiele für Bindungsmuster von anti-MUC1-Antikörpern gegenüber nicht glykosylierten MUC1-Peptiden verschiedener Länge (1-6 Tandem-Repeats).

**Beispiel 3: Bindung von MUC1-spezifischen Antikörpern an PDTR-glykosylierte multiple Tandem- Repeats**

Synthetische MUC1-Glykopeptide des Typs:

[AHGVTSAPDT(GalNAc)RPAPGSTAPPA]$_n$ ; n = 1-5
wurden in einer Konzentration von 0,5μg/Well in Wasser bei 4°C über Nacht auf einer 96-Well-TC-Mikrotestplatte durch Antrocknen immobilisiert. Die Platten wurden gewaschen (PBS mit 0,05 % Tween20) und anschließend für 2 h mit 50 μl/Well einer Lösung von 10μg/ml gereinigtem Antikörper in PBS/BSA bei 37 °C inkubiert. Nach dreimaligem Waschen wurde die Platte mit Peroxidase-markiertem polyklonalem Kaninchen-anti-Maus-Immunglobulin-Serum (Dako) in einer Verdünnung von 1:2000 für 1,5 h bei 37 °C inkubiert. Nach dreimaligem Waschen wurde die Platte mit Ortho-phenylendiamin entwickelt und die Farbreaktion mit 2,5 N Schwefelsäure gestoppt. Die OD wurde bei 492 nm gemessen.

Die Anzahl der Tandem-Repeats pro Ansatz wurde in den Experimenten dadurch konstant gehalten, dass von allen verglichenen Glykopeptiden gleiche Gewichtsmengen pro Well eingesetzt wurden.

Figur 3 zeigt 2 Beispiele für die Bindung von MUC1-spezifischen monoklonalen Antikörpern an glykosylierte MUC1-Peptide unterschiedlicher Länge (= unterschiedlicher Anzahl an Tandem-Repeats).

Tabelle 3:

Bindungsmuster monoklonaler anti-MUC1-Antikörper gegen das immundominante PDTR-Motiv: Bindungsmuster gegenüber am PDTR glykosylierten und nichtglykosylierten MUC1-Peptiden unterschiedlicher Länge (1-6 Tandem Repeats) in Abhängigkeit von der Art des Immunogens

Antikörper Bindungsmuster in Abhängigkeit von:

A. Immunogen: MUC1 aus Tumormaterial

| | Glykosylierung | | | | Länge | | | | Länge mit Glykosylierung |
|---|---|---|---|---|---|---|---|---|---|
| | GD-1 | GD-2 | iGD | GI | LD-1 | LD-2 | LD-3 | LI | |
| A76-A/C7 | + | | | | + | | | | + |
| VU-11 E2 | + | | | | + | | | | + |
| VU-11D1 | + | | | | + | | | | (+) |
| Ma552 | | + | | | + | | | | - |
| VU-3C6 | | + | | | + | | | | - |
| BC4E549 | | + | | | | + | | | (+) |
| VU-12E1 | | + | | | | + | | | (+) |
| VU-3D1 | | + | | | | | + | | (+) |
| b-12 | | + | | | | | + | | (+) |
| B27.29 | | + | | | | | + | | - |
| MF 06 | | + | | | | | + | | - |

B. Immunogen:

MUC1-Peptide (unglykosyliert) oder Präparationen aus Nicht-Tumormaterial

| | Glykosylierung | | | | Länge | | | | Länge mit Glykosylierung |
|---|---|---|---|---|---|---|---|---|---|
| | GD-1 | GD-2 | iGD | GI | LD-1 | LD-2 | LD-3 | LI | |
| VU-4H5 | | | + | | | + | | | + |
| HMPV | | | + | | | + | | | (+) |
| HMFG-1 | | | | + | | + | | | - |
| BC2 | | | | + | | + | | | - |
| VA2 | | | | + | | + | | | - |
| Mc5 | | | | + | | + | | | - |
| E29 | | | | + | | | + | | - |
| BC3 | | | | + | | + | + | | - |
| 214D4 | | | | + | | | + | | - |
| BCP8 | | | | + | | | + | | - |
| VA1 | | | | + | | | + | | - |
| C595 | | | | + | | | | + | - |

(fortgesetzt)

| | Glykosylierung | | | | Länge | | | | Länge mit Glykosylierung |
|---|---|---|---|---|---|---|---|---|---|
| | GD-1 | GD-2 | iGD | GI | LD-1 | LD-2 | LD-3 | LI | |
| BC4W154 | | + | | | | | | + | (+) |

Die Ergebnisse zeigen, dass die meisten Antikörper, die durch eine Immunisierung von Mäusen mit Tumormaterial erzeugt wurden, positiv abhängig von der zunehmenden Länge PDTR-glykosylierter MUC1-Glykopeptide sind. Dies entspricht einem additiven Effekt der Antikörper bezüglich der PDTR-Glykosylierung und der Länge mit multiplen Tandem Repeats. Eine Untergruppe (A76-A/C7, VU-11 E2, VU-11D1) zeigt einen besonders starken additiven Effekt in dem die Bindung der Antikörper an MUC1-Glykopeptide mit mehreren PDTR-glykosylierten Tandem Repeats im Vergleich zur Bindung an kurzen PDTR-glykosylierten MUC1-Glykopeptiden (wie in Beispiel 1) sowie im Vergleich zur Bindung an nicht glykosylierten MUC1-Peptiden mit mehreren Tandem Repeats (wie in Beispiel 2) deutlich bis um ein Mehrfaches erhöht ist (s. Abb.3A). Antikörper mit einem additiven Bindungseffekt weisen eine besonders hohe Affinität gegenüber natürlichem Tumor-MUC1 auf, das aus multiplen Tandem Repeats besteht.

Die Antikörper, die durch eine Immunisierung von Mäusen mit Nicht-Tumormaterial erzeugt wurden, und PDTR glykosylierungsunabhängig sind, zeigen in der Regel keinen additiven Effekt, auch dann nicht, wenn die Bindungen in Bezug auf nicht glykosylierte MUC1 Peptide längenaghängig sind (LD-3 und LD-2).

Abbildung 3B zeigt ein Beispiel für eine Antikörperbindung ohne additiven Effekt.

Antikörper VU-4H5 und HMPV, die eine iGD Bindung zeigen, zeigen einen additiven Effekt, der in Beispiel 4 als Sonderfall näher beschrieben ist.

**Beispiel 4: Bindung von MUC1-spezifischen Antikörpern der GID-Gruppe an PDTR glykosylierte Glykopeptide mit multiplen Tandem Repeats**

Die Methodik entsprach der unter Beispiel 3 beschriebenen.

Die Ergebnisse zeigen, dass die Bindung des Antikörpers VU-4H5 aus der GID-Gruppe abhängig von der Anzahl der Tandem-Repeats pro MUC1-Glykopeptid ist. Dieses Ergebnis ist überraschend, da dieser Antikörper an kurze DTR-glykosylierte MUC1-Peptide praktisch nicht bindet. Er bindet jedoch zunehmend besser an DTRPglykosylierte MUC1-Glykopeptide mit zunehmender Zahl an Tandem-Repeats. Dieser Antikörper zeigt einen ähnlich additiven Effekt wie in Beispiel 3 beschrieben und nimmt damit eine Zwischenstellung ein, und macht ihn ebenfalls im Sinne der Erfindung für die verschiedenen Anwendungen einsetzbar.

Abbildung 4 zeigt die Bindung des Antikörpers VU-4H5 an glykosylierte MUC1-Peptide unterschiedlicher Tandem-Repeat-Anzahl.

**Beispiel 5A) Herstellung von immunstimulatorischem MUC1, das das MUC1 Tumorepitop trägt, zur Vakzinierung**

<u>a) Identifizierung von humanen Zellen, die MUC1 mit dem Tumorepitop stark exprimieren</u>

ZR-75-1 Zellen (ATCC: CRL 1500) wurden in RPMI-Medium mit 10% FKS und 1% Glutamin bei 37 °C, 8% $CO_2$ und 95% Luftfeuchte kultiviert. Diese Zellen exprimieren bereits, verglichen mit anderen Zellinien, große Mengen des Proteins mit dem Tumorepitop auf der Zelloberfläche. Figur 5A zeigt immuncytochemische Färbungen der ZR-75-1 Zellen mit dem A76-A/C7 Antikörper. Die immuncytochemische Färbung wurde durchgeführt, indem Zellen auf einen Objektträger getropft wurden, welche dann durch Inkubation im Zellkultur-Inkubator in einer feuchten Kammer für 30 min und anschließend bei Raumtemperatur für 15 min auf dem Objektträger immobiliert wurden. Die Zellen wurden in 5% Formalin in PBS 5 min. fixiert. Nach dreimaligem Waschen mit PBS wurden die Zellen 1,5 h bei Raumtemperatur mit dem primären Antikörper, der in Form eines Hybridomakulturüberstandes 1:5 verdünnt wurde, inkubiert. Nach erneutem dreimaligem Waschen mit PBS würde der sekundäre Antikörper (an Cy3-gekoppelter anti-Maus IgG aus dem Kaninchen, Dianova) in einer 1:1000 Verdünnung dazugegeben und weitere 30 min bei Raumtemperatur inkubiert. Die Einbettung nach weiterem dreimaligem Waschen erfolgte in der Mowiol-Lösung (Calbiochem, Heidelberg, Deutschland). Als Negativkontrolle wurde der isotypische IgG Antikörper MOPC-21 (Sigma) eingesetzt. Die immuncytochemische Färbung der ZR-75-1 Zellen mit dem A76-A/C7 machte deutlich, daß die Zellpopulation nicht homogen in Bezug auf die Expression des Tumorepitops war (Fig. 5A1). Um die Expression zu erhöhen und um eine homogene A76-A/C7 positive ZR-75-1 Zellpopulation zu erhalten, wurden die A76-A/C7 positiven ZR-75-1 Zellen über Magnetobeads selektioniert und anschließend durch limitierte Verdünnung solche Klone kloniert, die eine besonders starke und homogene Expression von geeigneten MUC1-Molekülen tragen. Die Selektion erfolgte durch Bindung von $10^7$ Zellen and den A76-A/C7 Antikörper (50 $\mu$l des Hybridomakulturüberstandes) und an Magnetobeads gekoppelte anti-IgM-Antikörper sowie MS-Säulen von Miltenyi Biotec GmbH (Bergisch Gladbach, Deutschland) unter Standardbedingungen (MACS; siehe Produktbeschreibung zu MS-Säulen). Die selektionierten Zellen wurden durch limitierte Verdünnung kloniert, indem die Zellen auf einen Zelltiter von 150 bis 300 Zellen pro 15 ml Kulturmedium eingestellt werden. 100 μl dieser Verdünnung wird auf einer Mikrotiterplatte verteilt, so daß 1-2 Zellen pro Loch kultiviert werden. Diese Verteilung auf der Mikrotiterplatte wird im Mikroskop überprüft. Auf diese Art und Weise konnten ZR-75-1 Sublinien isoliert werden, die das Tumorepitop homogen und stärker exprimierten, wie die immuncytochemische Färbung in Figur 5A2 zeigt.

Figur 5B zeigt, daß das MUC1-Protein auch im Vergleich zu anderen Zelllinien (z.B.: MCF-7) in großen Mengen in das Zellkulturmedium abgegeben wird. Dies ist das Ergebnis von ELISA-Analysen mit dem HMFG-1 (Klon 1.10.F3, Beckman/Coulter) als Fänger-Antikörper und dem A76-A/C7 als Detektions-Antikörper (Figur 5B). Die Proben für diesen ELISA wurden gewonnen, indem $10^5$ Zellen pro ml Zellkulturmedium ausgesät wurden, 3 Tage unter Standardbedingungen (siehe oben) kultiviert wurden, anschließend jeden Tag ein Aliquout abgenommen wurde und der Zellkulturüberstand durch Zentrifugation vom Zellpellet getrennt wurde. 50 μl dieser Überstände wurden unverdünnt oder in den angegebenen Verdünnungen (Figur 5B) im ELISA eingesetzt. Der HMFG-1-A76-A/C7-Sandwich-ELISA wurde durchgeführt, indem Mikrotiterplatten mit dem Fänger-Antikörper HMFG-1, 1:250 in PBS verdünnt (keine Konzentrationsangaben des Herstellers), über Nacht in 50 μl PBS pro "Well" bei 4 °C beschichtet wurden. Anschließend wurden die beschichteten Platten zweimal mit 0,1% Tween 20 in PBS bei Raumtemperatur blockiert. Der Blockierungspuffer wurde entfernt und die Platten erneut dreimal mit Waschpuffer gewaschen, um dann 50 μl der Proben, unverdünnt oder in den angegebenen Verdünnungen mit Zellkulturmedium, in jedes "Well" zu geben und 1,5 h bei Raumtemperatur zu inkubieren. Als negative Kontrolle wurde der primäre Antikörper durch Zellkulturmedium oder 2% BSA, 0,1% Tween 20 in PBS ersetzt. Nach dreimaligem Waschen mit Waschpuffer wurde der biotinylierte, affinitätsgereinigte A76-A/C7 Antikörper in einer 1:500 Verdünnung dazugegeben und weitere 1 h bei Raumtemperatur inkubiert. Nach erneutem dreimaligem Waschen erfolgte die Bindung von an Meerrettich-Peroxidase (horseraddish-peroxidase) gekoppeltem Streptavidin (1:500 verdünnt) für 20 min bei Raumtemperatur. Abschließend wurden die Platten zweimal in Waschpuffer und einmal in PBS gewaschen. Die Färbereaktion erfolgte in 50 μl 50 mM Acetatpuffer pH 6,0 mit 1mg/ml TMB (3,3', 5,5' Tetramethylbenzidin, Stammlösung: 100 mg/ml in DMSO, Sigma) und 0,025% $H_2O_2$ (Sigma) für 20 min im Dunkeln bei Raumtemperatur. Die Farbreaktion wurde durch Zugabe von 25 μl 2,5 N Schwefelsäure (Endkonzentration 0,07 N) gestoppt und im ELISA-Reader bei 450 nm mit einem Referenzfilter von 620 nm vermessen.
Die Glykosylierung des sekretierten MUC wurde im ELISA mit Hilfe verschiedener Antikörper analysiert. Dabei wurde im Vergleich zu dem oben beschriebenen HMFG-1-A76-A/C7 Sandwich-ELISA der A76-A/C7 Antikörper gegen Antikörper ausgetauscht, die die Tumor-assoziierten Kohlenhydrate Tn (HB-1, Dako, Glostrup, Dänemark) oder TF (A78-G/A7, U. Karsten et al., 1995) mit hoher Affinität binden. Der Nachweis der Sekundärantikörper (HB-1 und A78-G/A7) erfolgte mit einem an POD-gekoppelten anti-Maus-IgM (1:5000 verdünnt). Die Farbreaktion wurde in 25 mM Zitronensäure, Phosphatpuffer pH 5,0 mit 0,04% $H_2O_2$ und 0,4 mg/ml o-Phenylendiamin (Sigma) für 20 min im Dunkeln bei Raumtemperatur durchgeführt, durch Zugabe von 2,5 N Schwefelsäure (Endkonzentration 0,07 N) gestoppt und im ELISA-Reader bei 490 nm mit einem Referenzfilter von 630 nm vermessen. Es konnten die Tumor-assoziierten Kohlenhydrat-Epitope Tn und TF nachgewiesen werden, letzteres nur nach Neuraminidase-Behandlungen des MUC1.

<u>b) Überexpression von sekretorischem tumorassoziiertem -MUC1-Molekülen</u>

Die Überexpression des sekretorischen MUC1 wird erreicht durch Integration der MUC1- cDNA, deren Expression unter Kontrolle eines starken Promoters steht (Figur 5C) in eine Stelle im Genom der ZR-75-1 Zellen, die besonders transkriptionsaktiv ist. Zunächst wurden die Zellen mit dem Recsite-Vektor transfiziert, der nach Integration der Recsite im Genom, was durch Antibiotika-Selektion erreicht wurde, eine stabile Expression des LacZ Gens vermittelt. Die stabil transfizierten Zellen wurden kloniert durch limitierte Verdünnung (siehe nächster Absatz) und diejenigen Zellen identifiziert, die die stärkste LacZ-Aktivität zeigten. Diese Zellen erlaubten die Integration der MUC1-cDNA mit dem CMV-Promoter über die Recsite in eine transkriptionsaktive Stelle im Genom (Figur 5D). Die MUC1-cDNA wurde durch RT-PCR aus ZR-75-1 Zellen amplifiziert und durch Restriktion in den Expressionsvektor kloniert.

*Zellklonierung durch limitierte Verdünnung*

Die Zellklonierung durch limitierte Verdünnung wird durchgeführt, indem die Zellen auf einen Zelltiter von 150 bis 300 Zellen pro 15 ml Kulturmedium eingestellt werden. 100 μl dieser Verdünnung wird auf einer Mikrotiterplatte verteilt, so dass 1-2 Zellen pro Loch kultiviert werden. Diese Verteilung auf der Mikrotiterplatte wird im Mikroskop überprüft.

<u>c) Reinigung des sekretorischen MUC1</u>

Das sekretorische MUC1 wurde aus mehreren Litern Zellkulturüberstand mit Hilfe einer A76-A/C7 Affinitätschromatographie-Säule gereinigt unter StandardBedingungen. Die Analyse des gereinigten Proteins ergab im SDS-Polyacrylamidgel mehrere Banden, die mit einem käuflich erhältlichen MUC1-Präparat co-migrierten (Figur 5E).

**Beispiel 5B) Einfluß der T-Zellaktivierung durch gereinigtes sekretorisches MUC1, das das MUC1-Tumorepitop trägt**

Mit Hilfe des mit A76-A/C7 gereinigten sezernierten tumorassoziierten MUC1 wurde mit angereicherten T-Zellen (siehe oben) zusammen mit bestrahlten PBLs inkubiert. T-Zellen und PBLs stammten von zwei verschiedenen Patienten, so dass eine allogene Reaktion hervorgerufen wurde, die mittels T-Zellproliferation in BrdU-Assay (siehe oben) nachgewiesen wurde. In Anwesenheit von gereinigtem sezerniertem tumorassoziiertem MUC1 wurden keinerlei Hinweise auf ein immunsuppressiven Effekt von MUC1 in diesen "Mixed Leukocyte Reaktion" festgestellt. Im Gegenteil, es konnte bei der höheren Konzentration ein deutlicher Stimulierungseffekt beobachtet werden. Referenzsubstanzen waren PBS beziehungsweise 10 $\mu$g/ml bovines submaxillarisches Mucin (BSM)(Figur 5F).

**Beispiel 6**

Herstellung eines monoklonalen Antikörper mit Hilfe von mit A76-/C7 gereinigtem immunstimulatorischen MUC1, der einen besonders starken additiven Bindungseffekt aufweist.

1.Herkunft der Ausgangszellen

a) Die Milzzellen wurden unter sterilen Bindungen (Laminarbox) aus einer gemäß 2. immunisierten weiblichen Maus des Inzuchtstammes Balb/c gewonnen.

b) Als Fusionszellinie kam X63-Ag8.653 (Plasmozytom-Zellinie von Balb/c; Kearney et al., J. Immunol 123:1548-1550, 1979) zur Anwendung. Die Haltung erfolgte in Medium RPMI 1640 unter Zusatz von 10% inaktiviertem fötalen Kälberserum und 5 x $10^{-5}$ M $\beta$-Merkaptoethanol.

c) Als Feederzellen dienten Peritonealzellen aus Balb/c-Mäusen der gleichen Herkunft wie unter a). Sie wurden unter sterilen Bedingungen nach einer publizierten Methodik am Vortag des Fusionsexperiments bzw. der Klonierung entnommen und in Zellkultur-Mikrotestplatten eingesät (Karsten, in: Friemel, Immunologische Arbeitsmethoden, 4. Aufl., Jena 1991, pp. 309-320).

2. Immunisierung

Als Immunogen diente 100 $\mu$g MUC1 das durch Affinitätschromatographie mit A76-A/C7 gewonnen wurde (siehe Beispiel 5). MUC1 wurde in PBS mit inkomplettem Freundschem Adjuvans i.p. appliziert, und 5 Wochen und 9 Wochen später jeweils mit dem A76-A/C7 gereinigten MUC1 geboostert.

3.Generierung der Hybridome

Die Hybridomtechnik wurde nach publizierten Standardmethoden durchgeführt (Peters et al., Monoklonale Antikörper, Herstellung und Charakterisierung, Berlin 1985). Die Fusion erfolgte 4 Tage nach der letzten Boosterung. Als fusogenes Agens wurde PEG benutzt. Die Selektion der Hybridome erfolgte mittels Azaserin/Hypoxanthin; zur Wachstumsunterstützung wurden Maus-Peritonealzellen verwendet.
Die entstehenden Hybridome wurden mittels ELISA an immobilisierten MUC1, MUC1 Glykopetiden und in Immunofluoreszenztests an MUC1 positiven Zelllinien (zum Beispiel T-47D-Zellen, ZR-75-1-Zellen und Subzelllinien) auf der Sekretion spezifischer Antikörper getestet.
Klonierungen erfolgten mittels limitierender Verdünnung nach Standardtechniken in Gegenwart von Maus-Peritonealzellen (Karsten, in: Friemel, Immunologische Arbeitsmethoden, 4. Aufl., Jena 1991, pp. 309-320). Die Isotypen wurden mittels eines kommerziellen Isotypen-ELISA-Kits (Pharmigen) ermittelt.

**Beispiel 7: Untersuchungen zur tumorspezifischen Zytotoxizität**

**a) Identifizierung und Genreierung einer Subpopulation von humanen MCF-7 Zellen, die MUC1 mit dem Tumorepitop stark exprimieren**

MCF-7 Zellen (ATCC: HTB-22) wurden, wie im Detail unter Beispiel 5 für ZR75-1 beschrieben, kultiviert und aus den Zellen mit Hilfe von Magnetobeads mit A76-A/C7 Zellen isoliert und anschließend durch limitierte Verdünnung solche Klone kloniert, die eine besonders starke und homogene Expression von geeigneten MUC1-Molekülen tragen. Auf diese Art und Weise konnten MCF-7 Sublinien isoliert werden, die das Tumorepitop homogen und stärker exprimierten als die Ausgangslinie.

**b) Generierung reifer Nemod-mDC Zellen beladen mit Lysaten aus MCF-7**

Als dendritische Zellen wurden effektive dendritische Zellen (DE 10139428.4) aus NemodDC-1 Zellen, die von MUTZ-3 abstammen, hergestellt. Effektive dendritische Zellen wurden durch Differenzierung aus NemodDC-1 Zellen (precursor Zellen, prec-NemodDC) zu unreifen dendritischen Zellen (i-NemodDC) differenziert, mit Antigen beladen und anschließend zu reifen effektiven dendritischen Zellen (m-NemodDC) gereift, die antigen-spezifisch T-Zellen aktivieren können. I-NemodDC, die mit Antigenen beladen werden, nehmen diese auf, prozessieren sie und präsentieren Peptidfragmente der Antigene im Kontext ihrer MHC-Klasse-Moleküle, beispielsweise MHC I-A2. Mit Hilfe dieser effektiven dendritischen Zellen können sowohl spezifische CTL- und Helfer-T-Zellen aus naiven T-Zellen stimuliert werden, als auch NKT-Zellen stimuliert und expandiert werden.
4 x $10^6$ prec-Nemod-DC Zellen wurden in 40 ml Reaktionsvolumen in $\alpha$MEM + 20 % FCS + 10 % CM5637 mit 1000 U/mL GM-CSF, 1000 U/mL IL-4 und 2,5 ng/mL TNF$\alpha$ für 7 Tage differenziert. Anschließend wurden die Zellen (i-NemodDC) für 6 h mit einer geeigneten Menge an Tumorzelllysat aus den oben beschriebenen MCF-7 Zellen, die durch mehrfaches abwechselndes Frieren und Tauen mit Hilfe von flüssigem Stickstoff hergestellt wurden, beladen und anschließend für weitere 24 h mit TNF$\alpha$ gereift, bestrahlt (30 Gy) und zur Stimulation von T-Lymphozyten eingesetzt.

**c) Isolierung von CD8+-T-Zellen mittels Microbeads**

CD8+-T-Zellen Zellen HLA-A2 positiver Spender wurden über eine MACS Isolierung mit Hilfe von Antikörperbeschichteten Magnetbeads (Miltenyi, Herstellerproduktinformation) nach bekannten Verfahren aus PBMC aus herkömmlichen Buffy Coat-Präparationen aufgereinigt.

**d) Generierung zytotoxischer T-Zellen (CTLs)**

Es wurden die CD8+ T-Lymphozyten (1 x $10^6$ CD8+-T-Lymphozyten/mL) eines HLA-A2 positiven Spenders wöchentlich mit Tumorzelllysat (MCF7 Lysaten) beladenen m-NemodDC (Ratio 1:10) in Medium (AIM V + 10 U/mL IL-2 ab Tag 7) stimuliert (Prime + Restimulation).

**e) Zytotoxizitätstest**

Zunächst wurden die Zielzellen (MCF-7, LS 174 T und T2), die sich in einem physiologisch stabilen und vitalem Zustand befinden, in kaltem Medium (DMEM + 5 % FCS) gewaschen und nach Zentrifugation (300 x g, 7 min, RT) in Europiumpuffer (50 mM HEPES, 93 mM Natriumchlorid, 5 mM Kaliumchlorid, 2 mM Magnesiumchlorid, 10 mM DTPA, 2 mM Europium-III-acetat) aufgenommen. Dabei wurden 5 x $10^6$ Zielzellen in einem Volumen von 800 $\mu$l in eine Elektroporationsküvette (d = 4 mm) überführt. Vor und nach der Elektroporation erfolgte eine 6 bis 10minütige Inkubationszeit auf Eis. Die Elektroporation wurde mit einem Eppendorf Multiporator (30 $\mu$s, 710 V oder 1100 V und 1 Puls) durchgeführt. Nach 5maligem Waschen mit Medium steht die markierte Zielzelle zum Einsatz im Zytotoxizitätstest zur Verfügung. Dabei wurde sie entweder nach der Bestimmung der Vitalität und Zelldichte direkt eingesetzt (MCF-7 oder LS 174 T) oder zur Untersuchung von Peptid-spezifischen Effektorzellen für weitere 2 Stunden mit Peptid (MUC1 A2-Peptid: z.B. LLLLTVLTL; irrelevantes HIV-gag A2-Peptid; jeweils 5$\mu$g/ml) markiert (T2 Zellen). Für den Zytotoxizitätstest wurden markierte Zielzellen (5 bis 10 x $10^3$) in einem Volumen von 100 $\mu$L/Vertiefung in einer Mikrotiterplatte mit Rundboden vorgelegt. Anschließend wurden Effektorzellen, ebenfalls in einem Volumen von 100 $\mu$L, in verschiedenen Zellkonzentrationen hinzugegeben, sodass ein Verhältnis der Effektorzelle zur Zielzelle von 1,25:1 bis 100:1 resultiert. Neben diesen Ansätzen wurden Proben zur Bestimmung des Hintergrundsignals (BR), der spontanen und maximalen Freisetzung angelegt. Das Hintergrundsignal bestand aus einer zellfreien Medienprobe zu Beginn des Versuches. Die spontane Freisetzung (SR) gibt eine Aussage über die Stabilität der Markierung der Zielzelle mit Europium. Zur Bestimmung wurden markierte Zielzellen ohne Effektorzellen kultiviert und nach Beendigung der Inkubationszeit die Konzentration des freigesetzten Europiums ermittelt. Die maximale Freisetzung (MR) wurde anhand von markierten Zielzellen bestimmt, die ebenfalls ohne Effektorzellen, jedoch mit einem künstlichen Reagenz (hier mit 50% reinstem Ethanol), über die Testdauer inkubiert und zur Totallyse gebracht wurden. Anhand der oben definierten Größen wurde der prozentuale Anteil der spontanen Freisetzung berechnet, der zur Auswertung eines erfolgreichen Tests unter 30% liegen sollte. Weiterhin wurde der prozentuale Anteil der spezifischen, zytotoxischen Aktivität der Effektorzelle charakterisiert.

$$\%spon\tan eFreisetzung = \frac{SR(Counts) - BR(Counts)}{MR(Counts - BR(Counts)} \times 100$$

$$\%spezifischeFreisetzung = \frac{Effektor\,/\,Zielzell(Counts) - SR(Counts)}{MR(Counts) - SR(Counts)} \times 100$$

Nach der Inkubationszeit von 4 Stunden wurde durch Zentrifugation der Mikrotiterplatte (500 x g, 5 min, RT) der europiumhaltige Kulturüberstand abgetrennt. Anschließend wurden 20 $\mu$l dieses Kulturüberstands in eine mit 200 $\mu$l Enhancement Lösung/Vertiefung vorgelegten Mikrotiterplatte mit Flachboden überführt und für weitere 15 min auf einem Plattenschüttler inkubiert. Während dieser Inkubationszeit entsteht aus dem Europium-DTPA Chelat unter Bildung eines Europium-NTA Chelats ein fluoreszierender Komplex. Die Proben wurden in einem zeitverzögerten Fluorometer (Victor$^2$, PerkinElmar), bei einer Wellenlänge von 613 nm gemessen.
In dem Versuch wurde MCF-7 als relevante Zielzelle (Figur 6, MCF7) und LS 174 T (Figur 6, LS174T) als eine irrelevante Zielzelle verwendet. Weiterhin wurden T2 Zellen, die nicht in der Lage sind zu prozessieren und nur durch definierte A2-Peptide auf ihren MHC Klasse I A2 Molekülen beladen werden können, verwendet. Hierbei wurde ein MUC1-A2-Peptid zum Test einer effektiven Stimulation von ursprünglich naiven T Zellen, die spezifisch gegen MUC1 gerichtet sind, mit Hilfe der erfindungsgemäßen MUC1-Moleküle, die im vorliegenden Beispiel in entsprechenden Zelllysaten vorhanden sind, verwendet (T2 + MUC1). Als Negativ-Kontrolle wurde ein irrelevantes HIVgag A2-Peptid verwendet (T2 + HIV).

Die Daten zeigen, dass mit Hilfe der erfindungsgemäßen MUC1-Moleküle in einem Zelllysat eine MUC1-spezifische cytotoxische Immunantwort gegen Zellen, die MUC1 auf der Oberfläche im Kontext von HLA-A2 tragen, erzeugt werden kann. Diese immunologischen Daten zeigen, dass die erfindungsgemäßen MUC1-Moleküle sowohl als solche zur Aktivierung einer effektiven Anti-Tumor-Antwort verwendet werden können als auch in Form von dendritischen Zellen, die mit dem erfindungsgemäßen MUC1-Molekülen beladen wurden. Außerdem zeigen die Daten, dass MUC1-spezifische T Zellen mit Hilfe der erfindungsgemäßen MUC1-Moleküle und dendritischen Zellen ex vivo expandiert und spezifisch aktiviert werden können, wodurch eine Eignung für adoptive T-Zelltherapien gezeigt wird.

## Patentansprüche

1. In vitro Verfahren zur Herstellung eines MUC1-Moleküls, welches in der Lage ist eine stimulatorische Immunantwort im Mensch hervorzurufen, umfassend:

   (a) in Kontakt bringen eines Gemisches von MUC1-Molekülen aus dem Überstand von ZR-75-1 Zellen mit dem Antikörper A76-A/C7
für einen Zeitraum, der ausreichend ist und unter Bedingungen, die geeignet sind, damit sich ein Immunkomplex ausbildet;
(b) Isolierung des Immunkomplexes; und
(c) Bereitstellung des MUC1-Moleküls aus dem Immunkomplex.

**2.** Verfahren nach Anspruch 1, wobei Affinitätschromatographie verwendet wird.

**3.** Verfahren zur Herstellung eines Arzneimittels umfassend die Schritte des Verfahrens nach Anspruch 1 oder 2 und weiterhin umfassend den Schritt der Formulierung des MUC1-Moleküls in pharmazeutisch verträglicher Form.

**4.** Verwendung eines immunstimulatorischen MUC1-Moleküls erhältlich durch ein Verfahren nach Anspruch 1 oder 2 zur Herstellung eines Arzneimittels zur Behandlung oder Prävention von Tumoren.

**5.** Immunstimulatorisches MUC1-Molekül erhältlich durch ein Verfahren nach Anspruch 1 oder 2 zur Verwendung bei der Behandlung oder Prävention von Tumoren.

**6.** Gereinigtes MUC1-Molekül, welches im Mensch immunstimulatorisch wirkt und erhältlich ist durch ein Verfahren nach Anspruch 1 oder 2.

## Claims

**1.** An in vitro method for the production of a MUC1 molecule which is able to generate a stimulatory immune response in a human comprising

(a) contacting a mixture of MUC1 molecules from the supernatant of ZR-75-1 cells with the antibody A76-A/C7 for a period of time which is sufficient and under conditions which are suitable so that an immune complex is formed;
(b) isolating the immune complex; and
(c) providing the MUC1 molecule from the immune complex.

**2.** The method of claim 1, wherein affinity chromatography is used.

**3.** A method for producing a pharmaceutical composition comprising the steps of the method of claim 1 or 2 and further comprising the step of formulating the MUC1 molecule in a pharmaceutically acceptable form.

**4.** Use of an immunostimulatory MUC1 molecule obtainable by a method of claim 1 or 2 for producing a pharmaceutical composition for the treatment or prevention of tumors.

**5.** Immunostimulatory MUC1 molecule obtainable by a method of claim 1 or 2 for use in the treatment or prevention of tumors.

**6.** Purified MUC1 molecule which has an immunostimulatory effect in humans and which is obtainable by a method of claim 1 or 2.

## Revendications

**1.** Procédé in vitro de fabrication d'une molécule MUC1 qui est capable de provoquer une réponse immunitaire stimulatrice chez l'homme, comprenant :

(a) amener en contact un mélange de molécules MUC1 provenant du surnageant de cellules ZR-75-1 avec l'anticorps A76-A/C7 pendant une période qui est suffisante et dans des conditions qui conviennent à la formation d'un complexe immun ;
(b) isoler le complexe immun ; et
(c) mettre à disposition la molécule MUC1 à partir du complexe immun.

2. Procédé selon la revendication 1, dans lequel une chromatographie d'affinité est utilisée.

3. Procédé de fabrication d'un médicament comprenant les étapes du procédé selon la revendication 1 ou 2 et comprenant en outre l'étape de la formulation de la molécule MUC1 sous forme pharmaceutiquement acceptable.

4. Utilisation d'une molécule MUC1 immunostimulatrice pouvant être obtenue par un procédé selon la revendication 1 ou 2 pour la fabrication d'un médicament pour le traitement ou la prévention de tumeurs.

5. Molécule MUC1 immunostimulatrice pouvant être obtenue par un procédé selon la revendication 1 ou 2 pour l'utilisation lors du traitement ou de la prévention de tumeurs.

6. Molécule MUC1 purifiée qui agit de manière immunostimulatrice chez l'homme et peut être obtenue par un procédé selon la revendication 1 ou 2.

Figur 1A

**Bindung des mAk A76-A/C7 (Typ GD-1) an ein MUC1 30-mer in Abhängigkeit von der Glykosylierung am PDTR-Motiv**

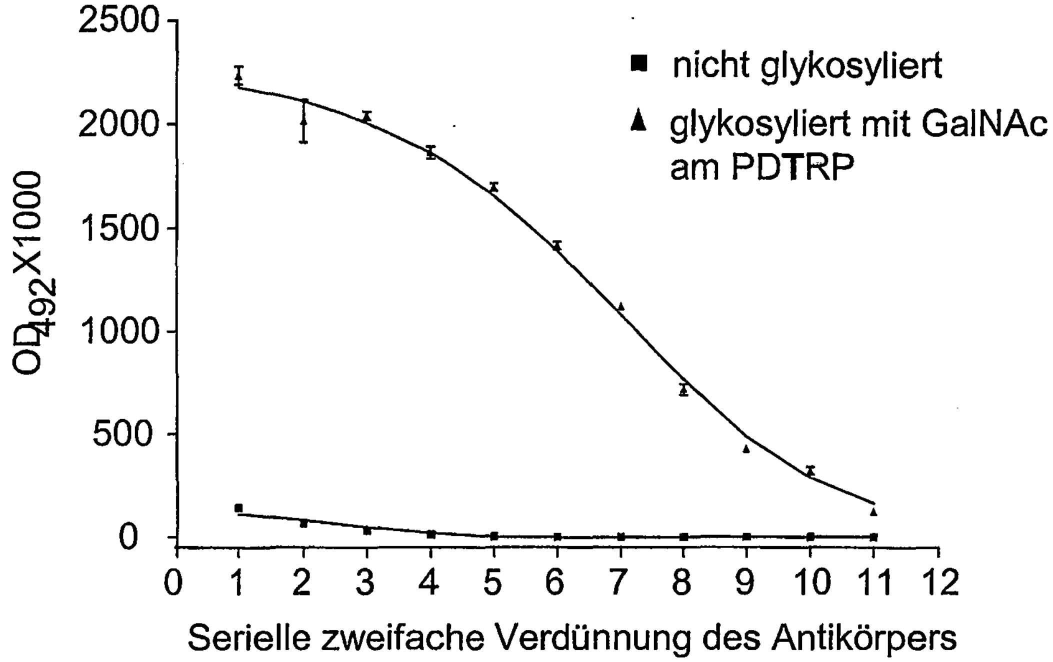


Figur 1B

**Bindungsmuster von mAk gegen MUC1 des Epitopes mit GalNAc: VU-3C6 (Typ GD-2), VU-4H5 (Typ iGD), HMFG-1 (Typ GI)**

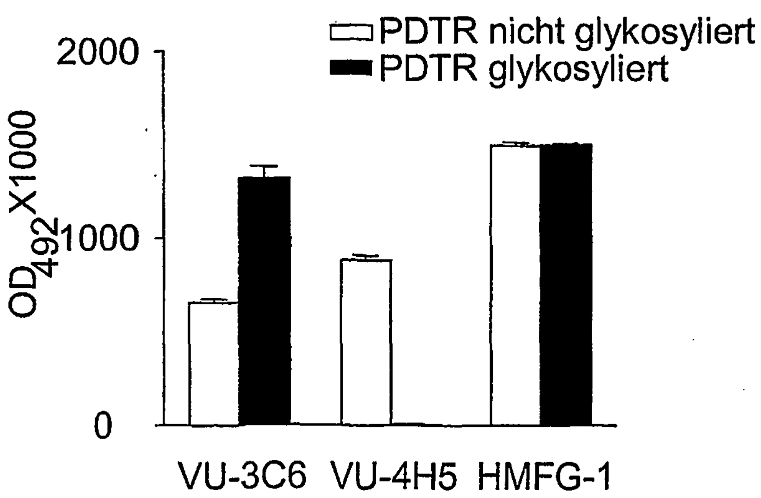

Figur 2

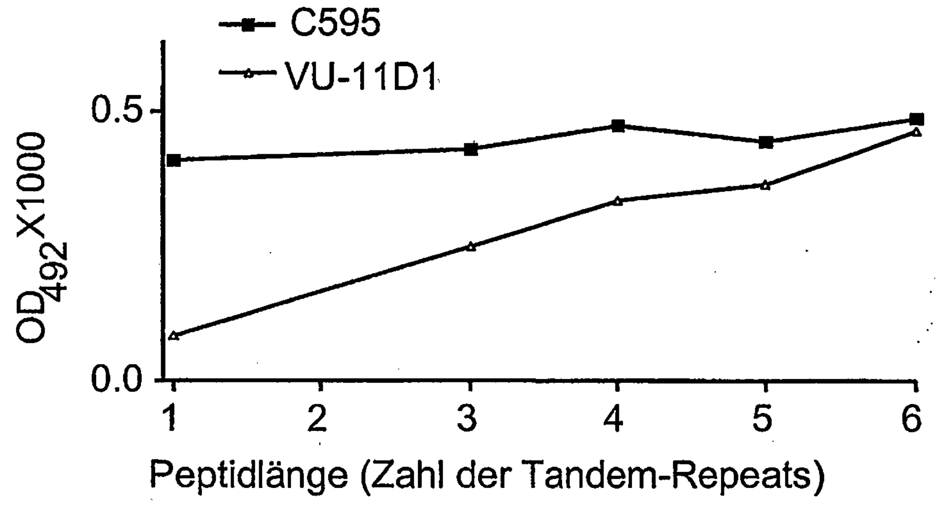
C595
VU-11D1
0.5
0.0
$OD_{492}$X1000
1
2
3
4
5
6
Peptidlänge (Zahl der Tandem-Repeats)

# Figur 3A

Bindung des mAk A76-A/C7 an glykosylierte MUC1-Peptide verschiedener Länge (1-5 Tandem Repeats) (glykosyliert mit Tn am PDTR-Motiv)

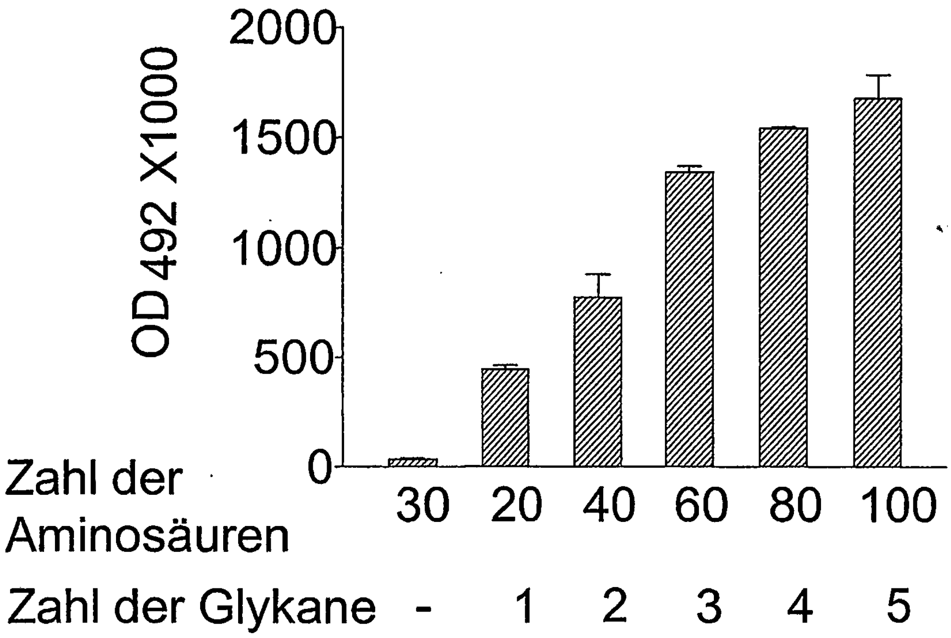

# Figur 3B

Bindung des mAk Mc5 an glykosylierte MUC1-Peptide verschiedener Länge (1-5 Tandem Repeats (glykosyliert mit Tn am PDTR-Motiv)

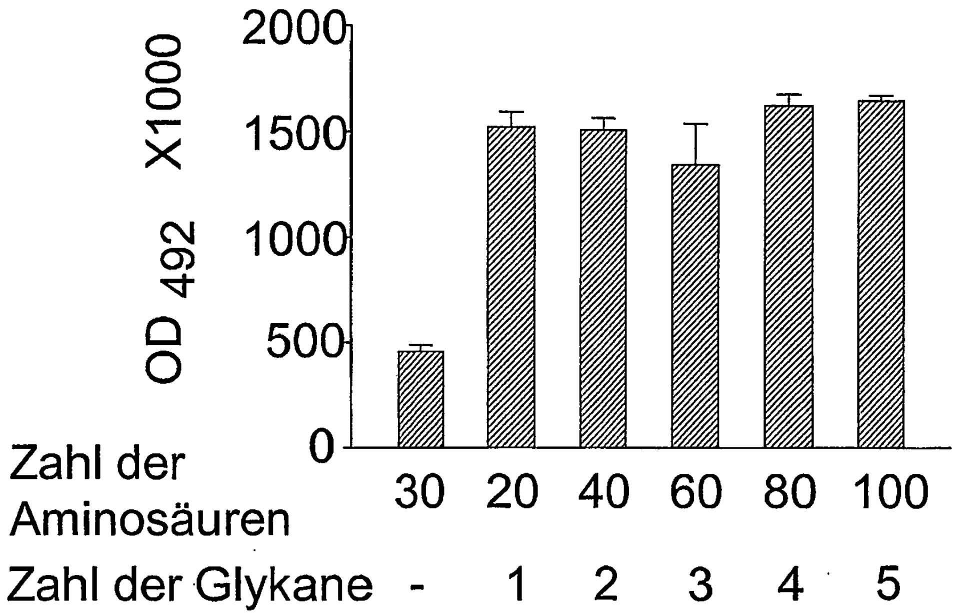

# Figur 4

Bindung des mAk VU-4H5 an glykosylierte MUC1-Peptide verschiedener Länge (1-5 Tandem Repeats) (glykosyliert mit Tn am PDTR-Motiv)

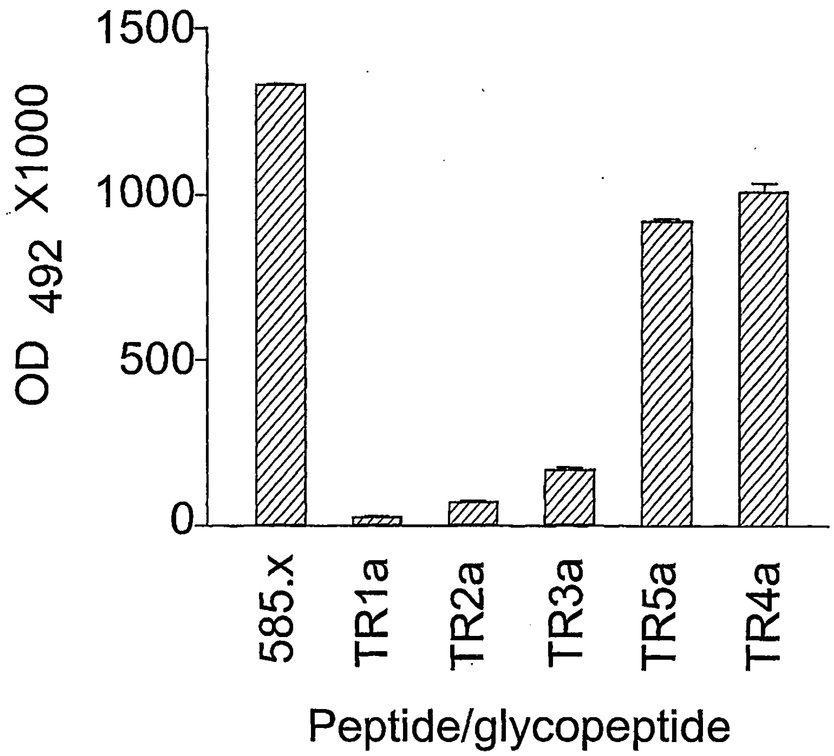

# Figur 5A

1) ZR-75-1 Zellen vor der Anreicherung durch A76-A/C7 und Klonierung

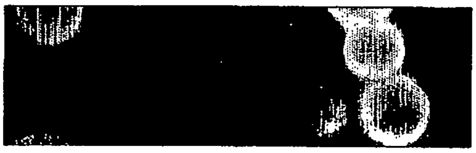

2) ZR-75-1 Zellen nach der Anreicherung durch A76-A/C7 und Klonierung (ZR-75-1-sub)

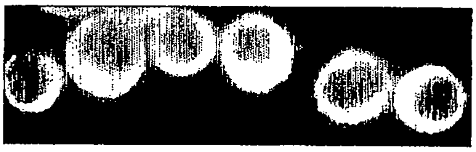

# Figur 5B

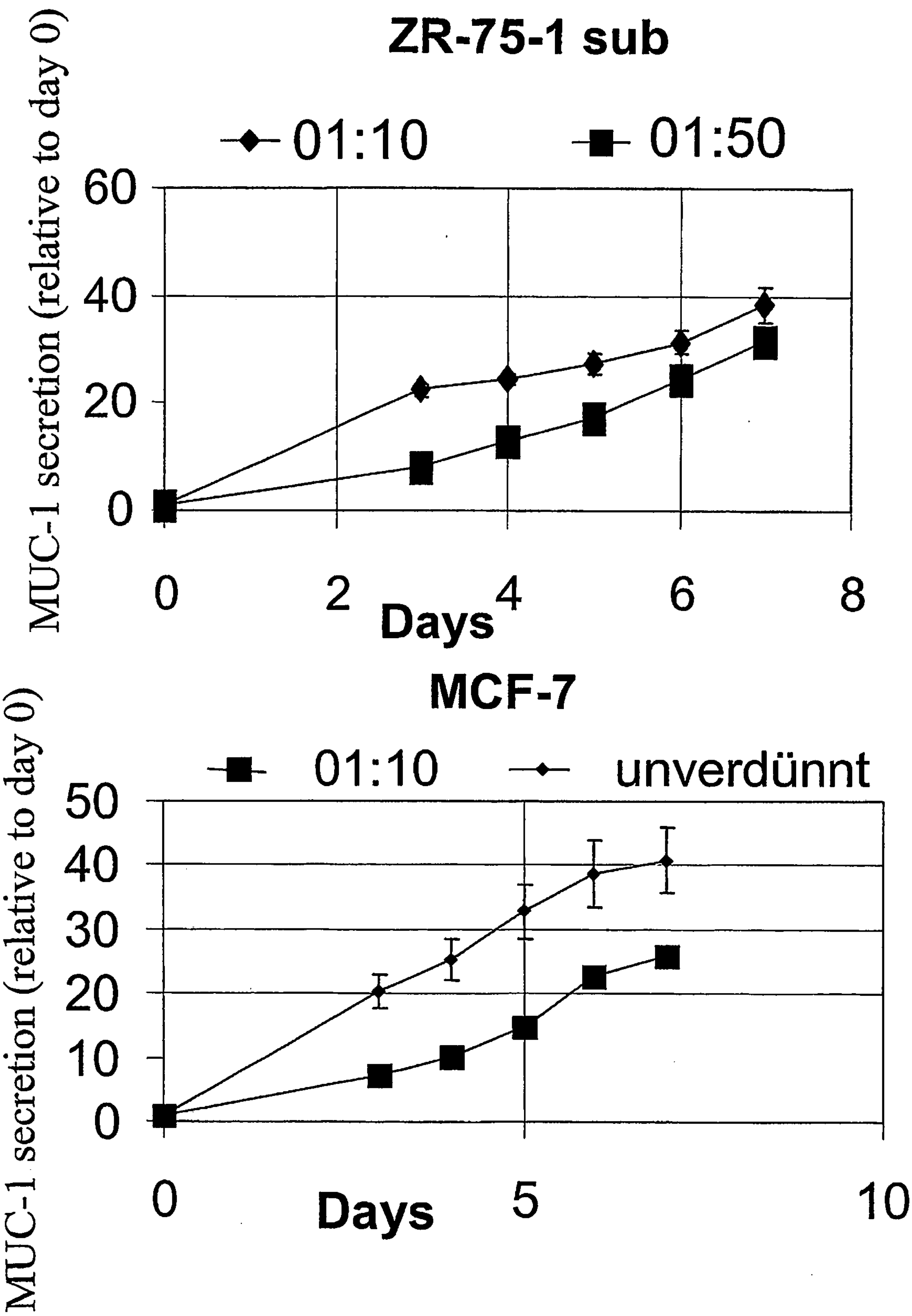
ZR-75-1 sub
01:10
01:50
MUC-1 secretion (relative to day 0)
60
40
20
0
0
2
4
6
8
Days
MCF-7
01:10
unverdünnt
MUC-1 secretion (relative to day 0)
50
40
30
20
10
0
0
5
10
Days

# Figur 5C (1)

A. Expressionsvektor

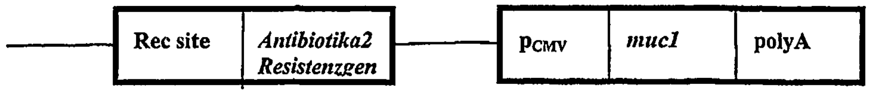


B. Sequenz der humanen muc1 cDNA, die in den Expressionsvektor kloniert wurden. Grün markiert sind die Primer, die zur Amplifizierung der cDNA aus ZR-75-1 cDNA verwendet wurden.

```
    GAATTCCCTG GCTGCTTGAA TCTGTTCTGC CCCCTCCCCA CCCATTTCAC
1   ---------- ---------- ---------- ---------- ----------
    CTTAAGGGAC CGACGAACTT AGACAAGACG GGGGAGGGGT GGGTAAAGTG
              +10        +20        +30        +40
    CACCACCATG ACACCGGGCA CCCAGTCTCC TTTCTTCCTG CTGCTGCTCC
51  -------*** ********** ********** ********** **********
    GTGGTGGTAC TGTGGCCCGT GGGTCAGAGG AAAGAAGGAC GACGACGAGG
              +10        +20        +30        +40
    TCACAGTGCT TACAGTTGTT ACAGGTTCTG GTCATGCAAG CTCTACCCCA
101 ********** ********** ********** ********** **********
    AGTGTCACGA ATGTCAACAA TGTCCAAGAC CAGTACGTTC GAGATGGGGT
              +10        +20        +30        +40
    GGTGGAGAAA AGGAGACTTC GGCTACCCAG AGAAGTTCAG TGCCCAGCTC
151 ********** ********** ********** ********** **********
    CCACCTCTTT TCCTCTGAAG CCGATGGGTC TCTTCAAGTC ACGGGTCGAG
              +10        +20        +30        +40
    TACTGAGAAG AATGCTGTGA GTATGACCAG CAGCGTACTC TCCAGCCACA
201 ********** ********** ********** ********** **********
    ATGACTCTTC TTACGACACT CATACTGGTC GTCGCATGAG AGGTCGGTGT
              +10        +20        +30        +40
    GCCCCGGTTC AGGCTCCTCC ACCACTCAGG GACAGGATGT CACTCTGGCC
251 ********** ********** ********** ********** **********
    CGGGGCCAAG TCCGAGGAGG TGGTGAGTCC CTGTCCTACA GTGAGACCGG
              +10        +20        +30        +40
    CCGGCCACGG AACCAGCTTC AGGTTCAGCT GCCACCTGGG GACAGGATGT
301 ********** ********** ********** ********** **********
    GGCCGGTGCC TTGGTCGAAG TCCAAGTCGA CGGTGGACCC CTGTCCTACA
              +10        +20        +30        +40
    CACCTCGGTC CCAGTCACCA GGCCAGCCCT GGGCTCCACC ACCCCGCCAG
351 ********** ********** ********** ********** **********
    GTGGAGCCAG GGTCAGTGGT CCGGTCGGGA CCCGAGGTGG TGGGGCGGTC
              +10        +20        +30        +40
    CCCACGATGT CACCTCAGCC CCGGACAACA AGCCAGCCCC GGGCTCCACC
401 ********** ********** ********** ********** **********
    GGGTGCTACA GTGGAGTCGG GGCCTGTTGT TCGGTCGGGG CCCGAGGTGG
              +10        +20        +30        +40
    GCCCCCCCAG CCCACGGTGT CACCTCGGCC CCGGACACCA GGCCGCCCCC
451 ********** ********** ********** ********** **********
    CGGGGGGGTC GGGTGCCACA GTGGAGCCGG GGCCTGTGGT CCGGCGGGGG
              +10        +20        +30        +40
    GGGCTCCACC GCCCCCCCAG CCCACGGTGT CACCTCGGCC CCGGACACCA
501 ********** ********** ********** ********** **********
    CCCGAGGTGG CGGGGGGGTC GGGTGCCACA GTGGAGCCGG GGCCTGTGGT
              +10        +20        +30        +40
    GGCCGCCCCC GGGCTCCACC GCGCCCGCAG CCCACGGTGT CACCTCGGCC
551 ********** ********** ********** ********** **********
    CCGGCGGGGG CCCGAGGTGG CGCGGGCGTC GGGTGCCACA GTGGAGCCGG
```

# Figur 5C (2)

```
                 +10        +20        +30        +40
       CCGGACACCA GGCCGGCCCC GGGCTCCACC GCCCCCCCAG CCCATGGTGT
601    ********** ********** ********** ********** **********
       GGCCTGTGGT CCGGCCGGGG CCCGAGGTGG CGGGGGGGTC GGGTACCACA
                 +10        +20        +30        +40
       CACCTCGGCC CCGGACAACA GGCCCGCCTT GGCGTCCACC GCCCCTCCAG
651    ********** ********** ********** ********** **********
       GTGGAGCCGG GGCCTGTTGT CCGGGCGGAA CCGCAGGTGG CGGGGAGGTC
                 +10        +20        +30        +40
       TCCACAATGT CACCTCGGCC TCAGGCTCTG CATCAGGCTC AGCTTCTACT
701    ********** ********** ********** ********** **********
       AGGTGTTACA GTGGAGCCGG AGTCCGAGAC GTAGTCCGAG TCGAAGATGA
                 +10        +20        +30        +40
       CTGGTGCACA ACGGCACCTC TGCCAGGGCT ACCACAACCC CAGCCAGCAA
751    ********** ********** ********** ********** **********
       GACCACGTGT TGCCGTGGAG ACGGTCCCGA TGGTGTTGGG GTCGGTCGTT
                 +10        +20        +30        +40
       GAGCACTCCA TTCTCAATTC CCAGCCACCA CTCTGATACT CCTACCACCC
801    ********** ********** ********** ********** **********
       CTCGTGAGGT AAGAGTTAAG GGTCGGTGGT GAGACTATGA GGATGGTGGG
                 +10        +20        +30        +40
       TTGCCAGCCA TAGCACCAAG ACTGATGCCA GTAGCACTCA CCATAGCACG
851    ********** ********** ********** ********** **********
       AACGGTCGGT ATCGTGGTTC TGACTACGGT CATCGTGAGT GGTATCGTGC
                 +10        +20        +30        +40
       GTACCTCCTC TCACCTCCTC CAATCACAGC ACTTCTCCCC AGTTGTCTAC
901    ********** ********** ********** ********** **********
       CATGGAGGAG AGTGGAGGAG GTTAGTGTCG TGAAGAGGGG TCAACAGATG
                 +10        +20        +30        +40
       TGGGGTCTCT TTCTTTTTCC TGTCTTTTCA CATTTCAAAC CTCCAGTTTA
951    ********** ********** ********** ********** **********
       ACCCCAGAGA AAGAAAAAGG ACAGAAAAGT GTAAAGTTTG GAGGTCAAAT
                 +10        +20        +30        +40
       ATTCCTCTCT GGAAGATCCC AGCACCGACT ACTACCAAGA GCTGCAGAGA
1001   ********** ********** ********** ********** **********
       TAAGGAGAGA CCTTCTAGGG TCGTGGCTGA TGATGGTTCT CGACGTCTCT
                 +10        +20        +30        +40
       GACATTTCTG AAATGTTTTT GCAGATTTAT AAACAAGGGG GTTTTCTGGG
1051   ********** ********** ********** ********** **********
       CTGTAAAGAC TTTACAAAAA CGTCTAAATA TTTGTTCCCC CAAAAGACCC
                 +10        +20        +30        +40
       CCTCTCCAAT ATTAAGTTCA GGCCAGGATC TGTGGTGGTA CAATTGACTC
1101   ********** ********** ********** ********** **********
       GGAGAGGTTA TAATTCAAGT CCGGTCCTAG ACACCACCAT GTTAACTGAG
                 +10        +20        +30        +40
       TGGCCTTCCG AGAAGGTACC ATCAATGTCC ACGACGTGGA GACACAGTTC
1151   ********** ********** ********** ********** **********
       ACCGGAAGGC TCTTCCATGG TAGTTACAGG TGCTGCACCT CTGTGTCAAG
                 +10        +20        +30        +40
       AATCAGTATA AAACGGAAGC AGCCTCTCGA TATAACCTGA CGATCTCAGA
1201   ********** ********** ********** ********** **********
       TTAGTCATAT TTTGCCTTCG TCGGAGAGCT ATATTGGACT GCTAGAGTCT
                 +10        +20        +30        +40
       CGTCAGCGTG AGTGATGTGC CATTTCCTTT CTCTGCCCAG TCTGGGGCTG
1251   ********** ********** ********** ********** **********
       GCAGTCGCAC TCACTACACG GTAAAGGAAA GAGACGGGTC AGACCCCGAC
```

## Figur 5C (3)

```
               +10        +20        +30        +40
    GGGTGCCAGG CTGGGGCATC GCGCTGCTGG TGCTGGTCTG TGTTCTGGTT
1301********** ********** ********** ********** **********
    CCCACGGTCC GACCCCGTAG CGCGACGACC ACGACCAGAC ACAAGACCAA
               +10        +20        +30        +40
    GCGCTGGCCA TTGTCTATCT CATTGCCTTG GCTGTCTGTC AGTGCCGCCG
1351********** ********** ********** ********** **********
    CGCGACCGGT AACAGATAGA GTAACGGAAC CGACAGACAG TCACGGCGGC
               +10        +20        +30        +40
    AAAGAACTAC GGGCAGCTGG ACATCTTTCC AGCCCGGGAT ACCTACCATC
1401********** ********** ********** ********** **********
    TTTCTTGATG CCCGTCGACC TGTAGAAAGG TCGGGCCCTA TGGATGGTAG
               +10        +20        +30        +40
    CTATGAGCGA GTACCCCACC TACCACACCC ATGGGCGCTA TGTGCCCCCT
1451********** ********** ********** ********** **********
    GATACTCGCT CATGGGGTGG ATGGTGTGGG TACCCGCGAT ACACGGGGGA
               +10        +20        +30        +40
    AGCAGTACCG ATCGTAGCCC CTATGAGAAG GTTTCTGCAG GTAATGGTGG
1501********** ********** ********** ********** **********
    TCGTCATGGC TAGCATCGGG GATACTCTTC CAAAGACGTC CATTACCACC
               +10        +20        +30        +40
    CAGCAGCCTC TCTTACACAA ACCCAGCAGT GGCAGCCACT TCTGCCAACT
1551********** ********** ********** ********** **********
    GTCGTCGGAG AGAATGTGTT TGGGTCGTCA CCGTCGGTGA AGACGGTTGA
               +10        +20        +30        +40
    TGTAGGGGCA CGTCGCCCTC TGAGCTGAGT GGCCAGCCAG TGCCATTCCA
1601*****----- ---------- ---------- ---------- ----------
    ACATCCCCGT GCAGCGGGAG ACTCGAGTCA CCGGTCGGTC ACGGTAAGGT
               +10        +20        +30        +40
    CTCCACTCAG GGCTCTCTGG GCCAGTCCTC CTGGGAGCCC CCACCACAAC
1651---------- ---------- ---------- ---------- ----------
    GAGGTGAGTC CCGAGAGACC CGGTCAGGAG GACCCTCGGG GGTGGTGTTG
               +10        +20        +30        +40
    ACTTCCCAGG CATGGAATTC C
1701---------- ---------- -
    TGAAGGGTCC GTACCTTAAG G
```

# Figur 5D (1)

A. Rec site Vektor

| | | | |
|---|---|---|---|
| $P_{SV40}$ | **ATG** | ***rec site*** | ***lacZ-antibiotika1 Resistenzgen*** |

**Merkmale der stabil transfizierten Wirtszellen:**

-Integration der Rekombinationsstelle in Verbindung mit dem lacZ-antibiotika1 Resistenz Fusionsgen
-Resistenz gegen das Antibiotikum 1, Expression des lacZ Gens (β-Galaktosidaseaktivität)
-in Abhängigkeit des Integrationsortes der Rekombinationsstelle und des Fusionsgenes im Chromosom unterscheiden sich die Transfektanten bzgl. der Stärke der Expression des Fusionsgens (chromosomaler Positionseffekt). Detektion durch unterschiedlich starke β-Galaktosidaseaktivitäten.

# Figur D (2)

A. Integration der muc1 cDNA über die Recsite in das ZR-75-1 Genom

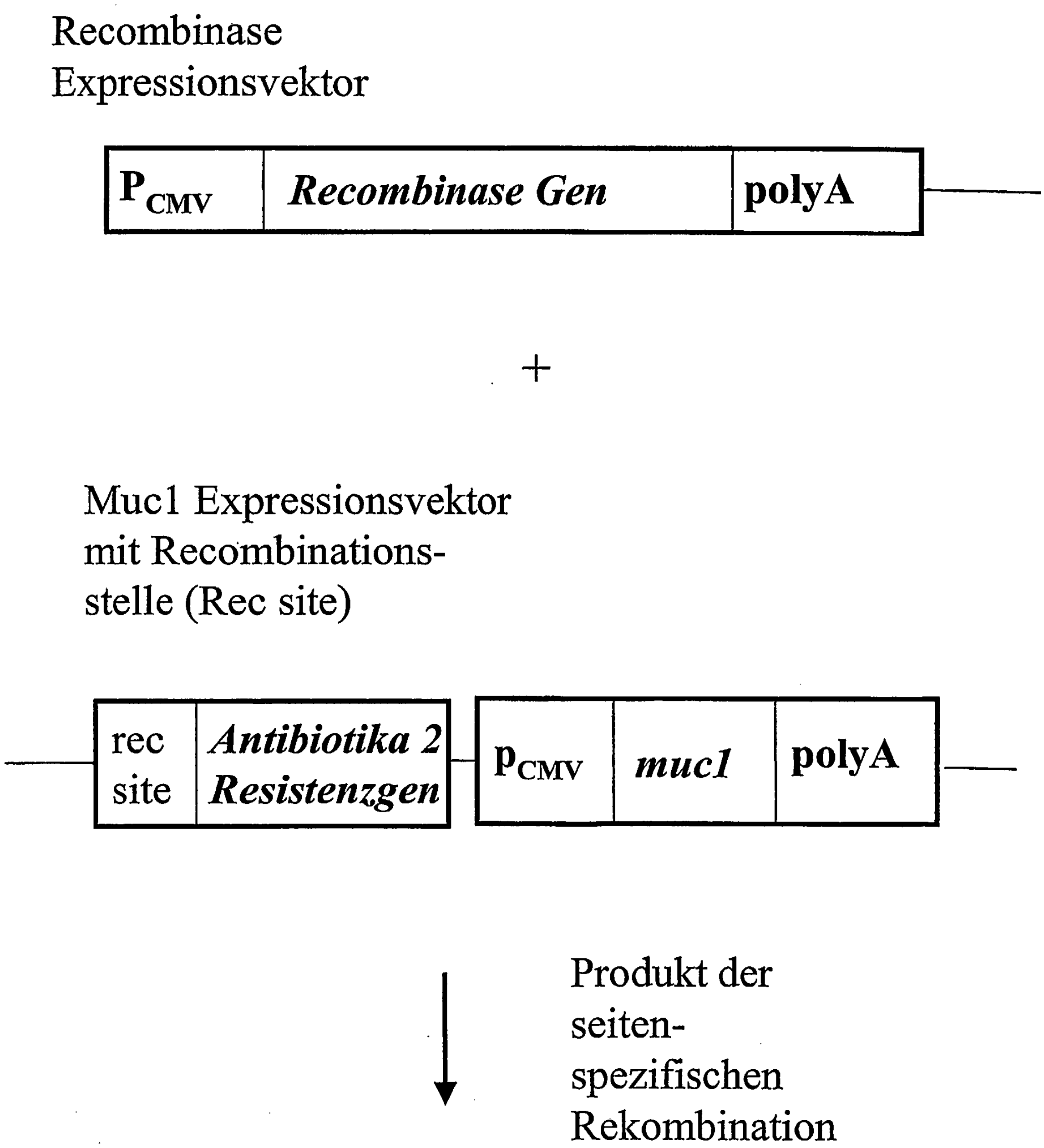

## Figur D (3)

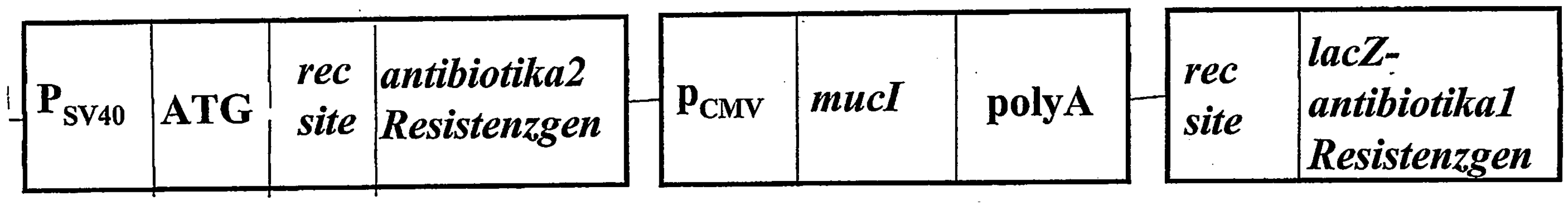


**Merkmale der stabil transfizierten Wirtzellen:**

- Resistenz gegen das Antibiotikum 2
- Sensitivität gegen das Antibiotikum 1, β- Galaktosidase inaktiv
- hohe Expression des recombinanten muc1 gens auf Grund des starken Promoters und des chromosomalen Positionsefekts

# Figur 5E

BSM - bovine submaxillary mucin 1
MUC1 – Mucin 1 aus Zellkulturüberständen

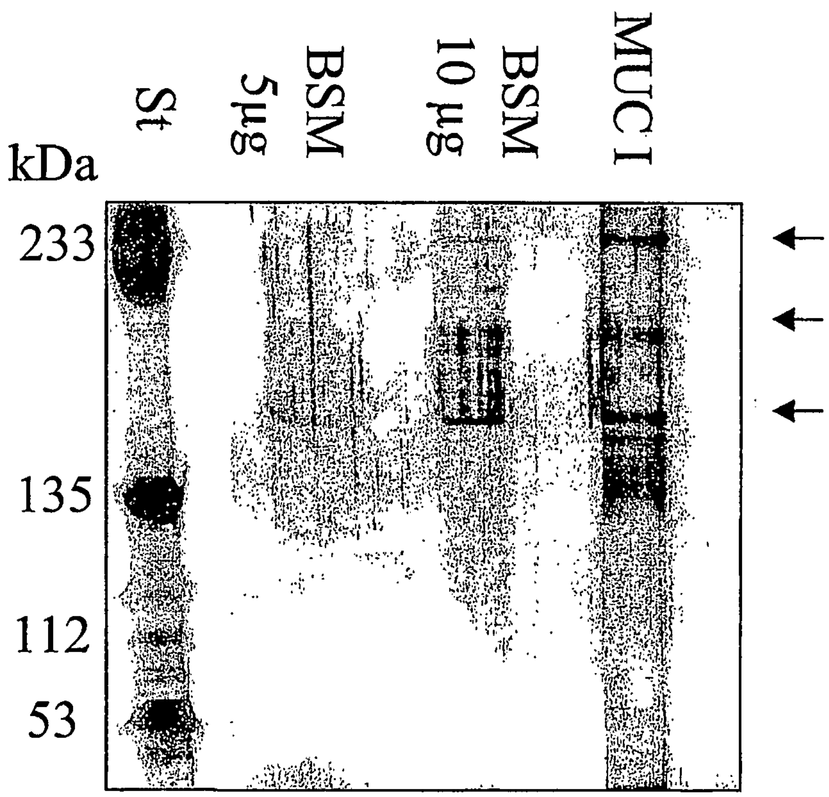

Figur 5F

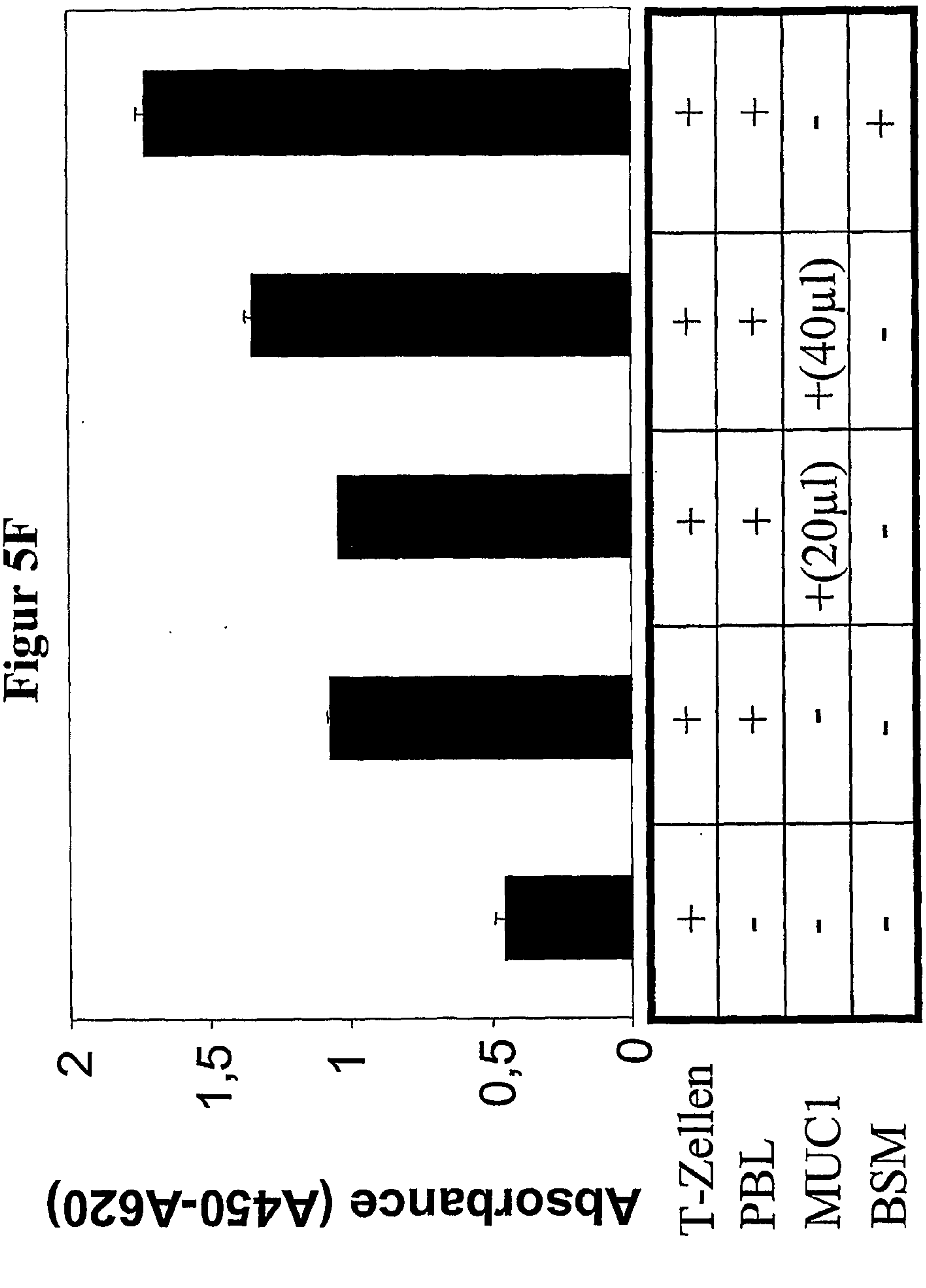
Absorbance (A450-A620)
2
1,5
1
0,5
0
T-Zellen + + + + +
PBL - + + + +
MUC1 - - +(20µl) +(40µl) -
BSM - - - - +

Figur 6

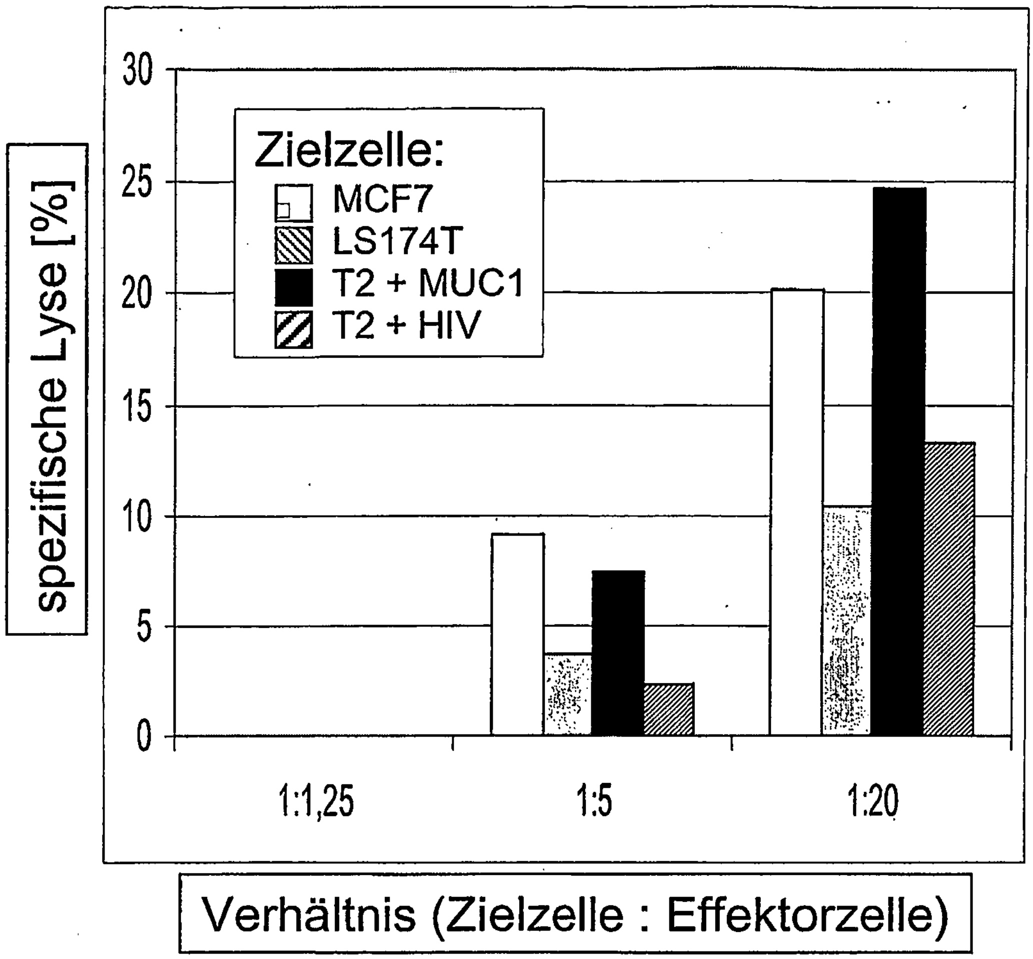


| | % SR |
|---|---|
| MCF7 | 8,86 |
| LS147T | 7,58 |
| T2 + MUC1 | 3,18 |
| T2 +HIV | 4,37 |

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9923114 A
- US 20020044943 A
- WO 9810783 A
- DE 10027695
- DE 19534630
- DE 10139428


### In der Beschreibung aufgeführte Nicht-Patentliteratur


- **FINN OJ ; JEROME KR ; HENDERSON RA ; PECHER G ; DOMENECH N ; MAGARIAN-BLANDER J ; BARRATT-BOYES SM.** MUC-1 epithelial tumor mucinbased immunity and cancer vaccines. *Imm Revs,* 1995, vol. 145 (61-89), 67
- **KARSTEN.** *Cancer Research,* 1998, vol. 58, 2541-2549
- **PRICE.** *Tumor Biol.,* 1998, vol. 1, 1-5
- **AGRAWAL et al.** *Nat. Med.,* Oktober 1998, vol. 4 (10), 1093
- **RYUKO et al.** *Tumor Biol,* 2000, vol. 21, 197-210
- **SNIJDEWINT et al.** *Cancer Immunol. Immunther,* 1999, vol. 48, 47-55
- **PRICE et al.** *Tumor Biol,* 1998, vol. 19 (I), 1-20
- **GIMMI et al.** *Nat. Med,* 1996, vol. 2 (12), 1367-1370
- **KEARNEY et al.** *J. Immunol,* 1979, vol. 123, 1548-1550
- **KARSTEN.** Friemel, Immunologische Arbeitsmethoden. Januar 1991, 309-320